# EUROPEAN PATENT SPECIFICATION

(11) **EP 3 984 355 B1**
(45) Date of publication and mention of the grant of the patent: **15.05.2024**
(21) Application number: 20202350.3
(22) Date of filing: 16.10.2020
(51) Int. Cl.: A01H 6/82, A01H 5/02

(54) **DOUBLE-FLOWERING DWARF CALIBRACHOA**
DOPPELT BLÜHENDE ZWERGKALIBRACHOA
CALIBRACHOA NAIN À DOUBLE FLORAISON

(43) Date of publication of application: 20.04.2022
(60) Divisional application: 23215531.7
(73) Proprietor: Klemm & Sohn GmbH & Co. KG, 70378 Stuttgart (DE)
(72) Inventor: Losert, Dominik, 71546 Aspach (DE); Klemm, Nils, 70378 Stuttgart (DE); Dohm, Andrea, 75177 Pforzheim (DE); Sander, Ulrich, 70378 Stuttgart (DE); Stöver, Anita, 73760 Ostfildern (DE)
(74) Representative: Wittop Koning, Tom Hugo

(56) References cited:
- US-A1- 2008 072 339
- SAKATA SEED CORPORATION: "Calibrachoa Cerv. ; SAKCAL108 ; sakcal108", COMMUNITY PLANT VARIETY OFFICE, CPVO, 3 BOULEVARD MARÉCHAL FOCH CS 10121 49101 ANGERS CEDEX 2 - FRANCE, 6 February 2014 (2014-02-06), XP090000666, [retrieved on 2018-09-21]
- SAKATA SEED CORPORATION: "Calibrachoa Cerv. ; SAKCAL105 ; sakcal105", COMMUNITY PLANT VARIETY OFFICE, CPVO, 3 BOULEVARD MARÉCHAL FOCH CS 10121 49101 ANGERS CEDEX 2 - FRANCE, 6 February 2014 (2014-02-06), XP090000663, [retrieved on 2018-09-21]
- DATABASE EMBL [Online] 31 March 2016 (2016-03-31), "Nicotiana sylvestris cultivar TW 137 mitochondrion, complete genome.", XP002802194, retrieved from EBI accession no. EM_STD:KT997964 Database accession no. KT997964

## Description

Double-flowering *Calibrachoa* varieties have been around since at least 2006, when the first Plant Patent Application was filed (PP18,694). Since then a number of double-flowering varieties have been developed, encompassing a wide range of colours and patterns. However as with all *Calibrachoa* varieties, these plants show high vigour and therefore plant growth regulators (PGR) need to be applied in order to achieve a more compact plant shape, which is commercially and economically desired. However, growth regulators are increasingly being banned by regulators. For instance, the growth regulator TILT^{®} is banned in the USA, Canada, Germany and Sweden.

Additionally, the timing of PGR application is very important for the shape of end products, but the correct moment of application is influenced by temperature and development stages of the plants. This means that it is difficult for a grower to apply PGR at the correct moment. Incorrect PGR application regularly leads to economic loss during cultivation. It is therefore also commercially interesting to breed *Calibrachoa* plants in which plants can be grown without PGRs.

Breeding of new plant varieties requires the continuous development of genetic diversity to obtain new, improved characteristics and traits. New genetic diversity can be established by crossing, random mutagenesis, or with the help of modern biotechnology.

The foregoing examples of the related art and limitations related therewith are intended to be illustrative and not exclusive. Other limitations of the related art will become apparent to those of skill in the art upon a reading of the specification.

### SUMMARY

The present disclosure describes a *Calibrachoa* plant comprising a double-flowering characteristic and a dwarf growth characteristic, wherein said double-flowering characteristic is caused by a mitochondrial allele associated with at least one single nucleotide polymorphism (SNP) mutation selected from the group consisting of a G to C nucleotide substitution at position number 320 of SEQ ID NO: 1 and an A to C nucleotide substitution at position number 247 in SEQ ID NO: 1, and wherein said dwarf growth characteristic is caused by a homozygous recessive nuclear allele associated with a SNP mutation consisting of a G to C nucleotide substitution at position 43 of SEQ ID NO: 2.

Accordingly, the present invention relates to a molecular marker for distinguishing a plant having the said allele for the said double flowering characteristic, said distinguishing comprising detecting at least one SNP mutation selected from the group consisting of 1.) a C nucleotide at position 320 of SEQ ID No:1 and 2) a C nucleotide at position number 247 in SEQ ID No:1, the marker comprising a sequence of at least 20 consecutive nucleotides of SEQ ID No:1, or the complementary sequence thereof, the said molecular marker preferably being isolated.

The present disclosure describes plants comprising double-flowering and dwarf growth characteristics, wherein the plant has a petaloid stamina rating of at least 2. At maturity the plant may have a vigour rating of less than 5 compared to plants having a G/C or G/G genotype at position 43 of SEQ ID NO: 2 when grown under the same environmental conditions. The plant may exhibit male sterility.

Described are plants comprising double-flowering and dwarf growth characteristics, wherein the plant is grown without the addition of synthetic plant growth regulators. The plant may comprise no detectable residue of a synthetic plant growth regulator or a related breakdown of a synthetic plant growth regulator product.

Described are plants comprising double-flowering and dwarf growth characteristics, wherein the plant further comprises a mutation affecting flower colour and/or flower colour pattern, wherein said mutation is the result of induced random or targeted mutagenesis. The targeted mutagenesis can be a gene editing tool or technology.

Further described is a method of producing a *Calibrachoa* plant comprising a double-flowering characteristic and a dwarf growth characteristic comprising the steps of crossing a first female *Calibrachoa* plant with a first male *Calibrachoa* plant to produce F₁ plants, wherein said first female *Calibrachoa* plant comprises a mitochondrial allele associated with at least one SNP mutation selected from the group consisting of a G to C nucleotide substitution at position number 320 of SEQ ID NO: 1 and an A to C nucleotide substitution at position number 247 in SEQ ID NO: 1 and exhibiting a double-flowering characteristic, and wherein said first male *Calibrachoa* plant has at least one copy of nuclear, recessive allele associated with a SNP mutation consisting of a G to C nucleotide substitution at position 43 of SEQ ID NO: 2, wherein when said nuclear allele is in the homozygous form, plants exhibit a dwarf growth characteristic; screening said F₁ plants for the presence of said nuclear SNP mutation; selecting an F₁ female plant exhibiting said double-flowering characteristic and further comprising at least one copy of said nuclear SNP mutation; crossing said F₁ female plant with said first male or a second male *Calibrachoa* plant having at least one copy of said nuclear SNP mutation to produce F₂ plants; screening said F₂ plants for the presence of said nuclear SNP mutation; and selecting an F₂ plant exhibiting said double-flowering characteristic and being homozygous for said nuclear SNP mutation.

The first or second male *Calibrachoa* plant can be homozygous for said nuclear SNP mutation and exhibits a dwarf growth characteristic. The described method may further comprise asexual propagation or sexual reproduction of the selected F₂ plant.

Also described are plants produced by the methods disclosed herein, wherein the plant produced is further asexually propagated and grown without synthetic growth regulators. The plant produced by the methods disclosed herein may have a petaloid stamina rating of at least 2 and a vigour rating of less than 5 at maturity when compared to plants having a non-dwarf growth characteristic when grown under the same environmental conditions, wherein said non-dwarf plant has at least one copy of the allele associated with a SNP mutation consisting of a G at position 43 of SEQ ID NO: 2 (i.e., having a G/C or G/G genotype at position 43 of SEQ ID NO: 2).

The plant produced by the methods disclosed herein may further comprise a mitochondrial allele associated with an A to C nucleotide substitution at position number 247 in SEQ ID NO: 1.

The plant produced by the methods disclosed herein may exhibit male sterility.

The plant produced by the methods disclosed herein may further comprise a mutation affecting flower colour and/or flower colour pattern.

Also described is a method for producing a double-flowering dwarf *Calibrachoa* plant having an additional desired trait comprising applying a plant breeding technique to the *Calibrachoa* plant produced by the methods disclosed herein. The plant breeding techniques may be selected from the group consisting of recurrent selection, mass selection, hybridization, open-pollination, backcrossing, pedigree breeding, mutation breeding, haploid/double haploid production, and marker enhanced selection. The plant breeding technique may be mutation breeding and the mutation selected may be spontaneous or artificially induced.

Also described is a plant produced by the methods disclosed herein, wherein said plant exhibits dwarf growth, double-flowering, and a desired trait. The desired trait may be flower colour and/or flower colour pattern.

As discussed above, the present disclosure relates to a molecular marker for distinguishing a plant having at least one allele for a double-flowering characteristic comprising at least one sequence selected from the group consisting of SEQ ID NO: 1, cDNA sequences thereof, fragments of at least 20 consecutive nucleotides thereof, and complementary sequences thereof.

In further embodiments, the present disclosure relates to a molecular marker for distinguishing a plant having at least one allele for a dwarf growth characteristic, said distinguishing comprising detecting a C nucleotide at position 43 of SEQ ID No:2, the marker comprising at least one sequence selected from the group consisting of SEQ ID NO: 2, cDNA sequences thereof, fragments of at least 20 consecutive nucleotides thereof, and complementary sequences thereof, said marker preferably being isolated,.

Also described is a molecular marker for distinguishing a plant having an allele for a double-flowering characteristic, comprising a sequence of at least 20 consecutive nucleotides of SEQ ID NO: 7, or the complementary sequence.

In some embodiments, the present disclosure teaches a method for distinguishing a plant having at least one allele for a double-flowering characteristic comprising using the corresponding molecular marker as described above and detecting at least one of a C nucleotide at position number 320 of SEQ ID NO: 1 and a C nucleotide at position number 247 in SEQ ID NO: 1. Detecting at least one of a C nucleotide at position number 320 of SEQ ID NO: 1 and a C nucleotide at position number 247 in SEQ ID NO: 1 may comprise obtaining genetic material; obtaining a nucleic acid, wherein said nucleic acid has at least a portion of sequence complementary to the molecular markers disclosed herein; and base-pairing said nucleic acid with said genetic material and examining the result of said base-pairing. The genetic material may be deoxyribonucleic acid, ribonucleic acid, or a combination thereof. The nucleic acid may be a primer set, a probe, or combination thereof.

In some embodiments, the present disclosure teaches a method for distinguishing a plant having at least one allele for a dwarf growth characteristic comprising using the corresponding molecular marker as described above, and detecting a C nucleotide at position 43 of SEQ ID NO: 2. Detecting a C nucleotide at position 43 of SEQ ID NO: 2 may comprise obtaining genetic material; obtaining a nucleic acid, wherein said nucleic acid has at least a portion of sequence complementary to the molecular markers disclosed herein; and base-pairing said nucleic acid with said genetic material and examining the result of said base-pairing. The genetic material may e.g. be deoxyribonucleic acid, ribonucleic acid, or a combination thereof. The nucleic acid may be a primer set, a probe, or combination thereof.

Also described is a plant distinguished by the markers and methods of using the markers disclosed herein, wherein the plant is homozygous for the allele for a dwarf growth characteristic, and wherein the plant is subsequently grown without synthetic growth regulators.

The following disclosures and aspects thereof are described and illustrated in conjunction with products and methods, which are meant to be exemplary and illustrative, not limiting in scope.

### BRIEF DESCRIPTION OF THE DRAWINGS

The patent or application file may contain one or more drawings executed in colour and/or one or more photographs.

The accompanying figures, illustrate some examples and/or features.
Figures 1A-1E shows photographs of flowers having different ratings (1-9) of flower filling (double-flowering), wherein 1 corresponds to single flowers and 9 corresponds to the highest level of flower filling. Figure 1A shows a flower having five single petals and a rating of 1. Figure 1B shows a flower having a double-flowering rating of 3. Figure 1C shows a flower having a double-flowering rating of 5. Figure 1D shows a flower having a double-flowering rating of 7. Figure 1E shows a flower having a double-flowering rating of 9.
Figure 2A corresponds to SEQ ID NO: 1 and shows the results of the Competitive Allele Specific PCR (KASP) assay of a single-flowering trait and the related sequence with the corresponding double-flowering trait. Two polymorphisms were identified in the double-flowering trait; an A to C nucleotide substitution at position number 247 and a G to C nucleotide substitution at position number 320, both indicated by bond, underlined font within brackets.
Figure 2B is a boxplot of the level of flower filling (y-axis) of genotypes having a C at position number 320 of SEQ ID NO: 1 compared to those having a G at position 320 of SEQ ID NO: 1 (x-axis). The validation of the findings within the SBG approach (turquoise box plots) is displayed in Figure 2B with additional box plots for the KASP assay (red box plots). The number within the boxplot indicates the number of genotypes in each group.
Figure 3 is a photograph of nine plants corresponding to the 1-9 rating scale for vigour (level of compactness), wherein a rating of 1 corresponds to the smallest, most compact plant. In the foreground, from left to right, are plants exhibiting ratings of 7, 8, and 9, respectively. The middle row, from left to right, shows plants exhibiting ratings of 4, 5, and 6, respectively. In the background, from left to right, are plants exhibiting ratings of 1, 2, and 3, respectively. All plants shown are the same age.
Figure 4A corresponds to SEQ ID NO: 2 and shows the results of the Competitive Allele Specific PCR (KASP) assay of wild-type and the related sequence with the corresponding dwarf trait. A G to C polymorphism was identified at position 43 of SEQ ID NO: 2 indicated by bond, underlined font within brackets.
Figure 4B is a boxplot of the level of growth vigour (y-axis) of genotypes carrying different allele composition for the detected nuclear "dwarf" polymorphism (x-axis) represented by SEQ ID NO: 2 (see also Figure 4A). The validation of the findings within the SBG approach (turquoise box plots) is displayed in Figure 4B with additional box plots for the KASP assay (red box plots). The number within the boxplot indicates the number of genotypes in each group.

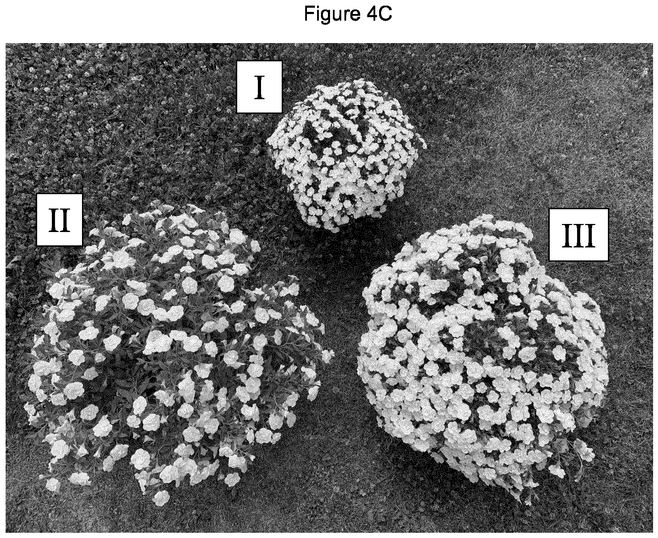

Figure 4C shows three *Calibrachoa* plants of two different genotypes. *Calibrachoa* plant I is a genotype with a combination of the double-flowering trait (SEQ ID NO: 1; "C") and a dwarf growth trait (SEQ ID NO: 2; "C/C"), not treated with any plant growth regulators and has a vigour rating of 3. *Calibrachoa* plants II and III demonstrate the same genotype with the double-flowering trait (SEQ ID NO: 1; "C") but do not comprise the dwarf growth trait (SEQ ID NO: 2; "G/G"). Plant II is untreated with any plant growth regulators and has a vigour rating of 7. Plant III was growth inhibited according to good horticultural practice (5 treatments of Dazide/B-Nine) and has a vigour rating of 6.
Figure 5 represents an example breeding scheme to produce plants having a double-flowering dwarf phenotype.
Figure 6 represents another example breeding scheme to produce plants having a double-flowering dwarf phenotype.
Figure 7 is a photograph of parental lines and progeny plants from a breeding scheme to produce a purple double-flowering dwarf *Calibrachoa* plant.
Figure 8 is a photograph of parental lines and progeny plants from a breeding scheme to produce a white double-flowering dwarf *Calibrachoa* plant.
Figures 9A-9G show plants of different colours all having the double-flowering trait combined with either a wild-type allele for growth, (plant pictured left, "G/G" or "G/C"), or homozygous for the recessive dwarf allele associated with the nucleotide polymorphism shown in Figure 4A (plant pictured right, "C/C") . Shown in Figure 9A are yellow varieties. Shown in Figure 9B are red varieties. Shown in Figure 9C are pink varieties. Shown in Figure 9D are pink-purple varieties. Shown in Figure 9E are purple-red varieties.
Figures 10A-10F are photographs of double-flowering dwarf *Calibrachoa* plants of the present disclosure having varying shades of white coloured flowers (Figure 10A and 10B), yellow coloured flowers (Figure 10C and 10D) and yellow-orange coloured flowers (Figure 10E and 10F).
Figures 11A-11F are photographs of double-flowering dwarf *Calibrachoa* plants of the present disclosure having varying shades of orange coloured flowers (Figure 11A and 11B) and red coloured flowers (Figures 11C - 11F).
Figures 12A-12F are photographs of double-flowering dwarf *Calibrachoa* plants of the present disclosure having varying shades of pink coloured flowers.
Figures 13A-13F are photographs of double-flowering dwarf *Calibrachoa* plants of the present disclosure having varying shades of pink coloured flowers.
Figures 14A-14D are photographs of double-flowering dwarf *Calibrachoa* plants of the present disclosure having varying shades of pink coloured flowers.
Figures 15A-15F are photographs of double-flowering dwarf *Calibrachoa* plants of the present disclosure having varying shades of purple coloured flowers.
Figures 16A-16F are photographs of double-flowering dwarf *Calibrachoa* plants of the present disclosure having patterns in different main and secondary coloured flowers.
Figures 17A-17F are photographs of double-flowering dwarf *Calibrachoa* plants of the present disclosure having different coloured flowers with contrasting veins and flowers with colour change during growing season.
Figures 18A-18F are photographs of double-flowering dwarf *Calibrachoa* plants of the present disclosure having different main coloured flowers and variations of secondary flower colour distribution.
Figures 19A-19F are photographs of double-flowering dwarf *Calibrachoa* plants of the present disclosure having different main coloured flowers and variations of secondary flower colour distribution.
Figures 20A-20C are photographs of double-flowering dwarf *Calibrachoa* plants of the present disclosure having different main coloured flowers and variations of secondary flower colour distribution.
Figures 21A-21F are photographs of double-flowering dwarf *Calibrachoa* plants of the present disclosure having different main coloured flowers showing variations of secondary flower colour distribution and flowers with colour change during growing season.

### DEFINITIONS

While the following terms are believed to be well understood by one of ordinary skill in the art, the following definitions are set forth to facilitate explanation of the presently disclosed subject matter.

All technical and scientific terms used herein, unless otherwise defined below, are intended to have the same meaning as commonly understood by one of ordinary skill in the art. References to techniques employed herein are intended to refer to the techniques as commonly understood in the art, including variations on those techniques and/or substitutions of equivalent techniques that would be apparent to one of skill in the art.

Following long-standing patent law convention, the terms "a," "an," and "the" refer to "one or more" when used in this application, including the claims. For example, the phrase "a cell" refers to one or more cells, and can refer to a tissue and/or an organ. Similarly, the phrase "at least one", when employed herein to refer to an entity, refers to, for example, 1, 2, 3, 4, 5, 6, 7, 8, 9, 10, 15, 20, 25, 30, 35, 40, 45, 50, 75, 100, or more of that entity, including but not limited to all whole number values between 1 and 100 as well as whole numbers greater than 100.

Unless otherwise indicated, all numbers expressing quantities of ingredients, reaction conditions, and so forth used in the specification and claims are to be understood as being modified in all instances by the term "about." The term "about," as used herein when referring to a measurable value such as an amount of mass, weight, time, volume, concentration or percentage is meant to encompass variations of ±20%, ±10%, ±5%, ±1%, ±0.5%, and ±0.1% from the specified amount, as such variations are appropriate to perform the disclosed methods and/or employ the disclosed compositions, nucleic acids, polypeptides, etc. Accordingly, unless indicated to the contrary, the numerical parameters set forth in this specification and attached claims are approximations that can vary depending upon the desired properties sought to be obtained by the presently disclosed subject matter.

As used herein, the term "and/or" when used in the context of a list of entities, refers to the entities being present singly or in combination. Thus, for example, the phrase "A, B, C, and/or D" includes A, B, C, and D individually, but also includes any and all combinations and subcombinations of A, B, C, and D (e.g., AB, AC, AD, BC, BD, CD, ABC, ABD, and BCD). One or more of the elements to which the "and/or" refers can also individually be present in single or multiple occurrences in the combinations(s) and/or subcombination(s).

As used herein, the phrase "an allele associated with [a particular SNP mutation or mutation]" can mean the actual causative mutation of the phenotype, or a linkage - either physical or functional, for example as a regulatory element or some sort of epistatic relationship. It is a recognizable and/or assayable relationship between two entities. Additionally, the SNP mutation or mutation "associated with" the allele serves as an indicator of whether the allele is present in a plant/germplasm and can be used to predict whether a plant is homozygous or heterozygous for the allele.

As used herein, the phrase "dwarf" as it relates to *Calibrachoa* refers to a plant phenotype (or trait or characteristic) caused by a homozygous recessive nuclear allele associated with a C nucleotide at position 43 of SEQ ID NO: 2. A "non-dwarf" plant would have at least one copy of an allele associated with a G nucleotide at position 43 of SEQ ID NO: 2. The phrase "dwarf" further means that the size of the mature plant is preferably less than 50% of the growth of a wild type Calibrachoa plant, more preferably less than 40%, and most preferably less than 30%, grown under the same conditions.

As used herein, the phrase "flower filling" or "filled" refers to the degree of the double-flowering characteristic and is measured herein according to UPOV's scale of 1-9 for quantitative traits.

"Genotype" as used herein refers to the genetic constitution of an individual organism.

As used herein, the term "human-induced mutation" or "induced mutagenesis" refers to any mutation that occurs as a result of either direct or indirect human action. This term includes, but is not limited to, mutations obtained by any method of targeted or human-induced random mutagenesis including for example, irradiation and treatment with mutation-inducing chemicals.

The term "isolated" means that the material is removed from its original environment (e.g., the natural environment if it is naturally occurring). For example, a naturally occurring nucleic acid or polypeptide present in a living plant is not isolated, but the same nucleic acid or polypeptide, separated from some or all of the co-existing materials in the natural system, is isolated. Such a nucleic acid could be part of a vector and/or such nucleic acid or polypeptide could be part of a composition (e.g, a cell lysate), and still be isolated in that such vector or composition is not part of the natural environment for the nucleic acid or polypeptide.

"mtC320" as used herein refers to the G to C nucleotide substitution (SNP mutation) at position number 320 of SEQ ID NO: 1 in the mitochondrial genome of a *Calibrachoa* plant.

"mtC247" as used herein refers to the A to C nucleotide substitution (SNP mutation) at position number 247 of SEQ ID NO: 1 in the mitochondrial genome of a Calibrachoa plant.

As used herein, the term "nucleotide sequence identity" refers to the presence of identical nucleotides at corresponding positions of two polynucleotides. Readily available sequence comparison and multiple sequence alignment algorithms are, respectively, the Basic Local Alignment Search Tool (BLAST) and ClustalW/ClustalW2/Clustal Omega programs available on the Internet (e.g., the website of the EMBL-EBI). Other suitable programs include, but are not limited to, GAP, BestFit, Plot Similarity, and FASTA, which are part of the Accelrys GCG Package available from Accelrys, Inc. of San Diego, Calif., United States of America. See also Smith & Waterman, 1981; Needleman & Wunsch, 1970; Pearson & Lipman, 1988; Ausubel et al., 1988; and Sambrook & Russell, 2001. One example of an algorithm that is suitable for determining percent sequence identity and sequence similarity is the BLAST algorithm, which is described in Altschul et al., 1990. Unless otherwise noted, alignments disclosed herein utilized ClustalW.

As used herein, the term "plant" can refer to a whole plant, any part thereof, or a cell or tissue culture derived from a plant. Thus, the term "plant" can refer to any of whole plants, plant components or organs (e.g., leaves, stems, roots, etc.), plant tissues, seeds and/or plant cells.

A plant cell is a cell of a plant, taken from a plant, or derived through culture from a cell taken from a plant. Thus, the term "plant cell" includes without limitation cells within seeds, suspension cultures, embryos, meristematic regions, callus tissue, leaves, shoots, gametophytes, sporophytes, pollen, and microspores. The phrase "plant part" refers to a part of a plant, including single cells and cell tissues such as plant cells that are intact in plants, cell clumps, and tissue cultures from which plants can be regenerated. Examples of plant parts include, but are not limited to, single cells and tissues from pollen, ovules, leaves, embryos, roots, root tips, anthers, flowers, fruits, stems, shoots, and seeds; as well as scions, rootstocks, protoplasts, calli, and the like.

"Phenotype" as used herein refers to the observable characteristics or traits of an organism that are produced by the interaction of the genotype and the environment.

The term "trait" or "characteristic" as used herein refers to any phenotypical characteristic including but not limited to those which are used to determine distinctness of a Calibrachoa variety in the context of plant variety protection. Relevant characteristic are known to the person skilled in the art and described - for example - in the "Guidelines for the conduct of test for distinctness, uniformity, and stability for Calibrachoa and Calibrachoa Cerv. (UPOV Code: CALIB) UPOV document TG/207/2 (Date 2016-03-16) of the International Union for the Protection of New Varieties of Plants (UOPV)", available under https://www.upov.int/edocs/tgdocs/en/tg207.pdf.

As used herein, "vigour" relates to overall plant size as measured by their potential to produce biomass and is rated herein according to UPOV's scale of 1-9 for quantitative traits.

### DETAILED DESCRIPTION

The following description includes information that may be useful in understanding the present disclosure.

### Overview

Described herein are *Calibrachoa* plants comprising a double-flowering characteristic and a dwarf growth characteristic, wherein said double-flowering characteristic is caused by a mitochondrial allele associated with at least one single nucleotide polymorphism (SNP) mutation of a G to C nucleotide substitution at position number 320 of SEQ ID NO: 1 and an A to C nucleotide substitution at position number 247 in SEQ ID NO: 1, and wherein said dwarf growth characteristic is caused by a homozygous recessive nuclear allele associated with a SNP mutation consisting of a G to C nucleotide substitution at position 43 of SEQ ID NO: 2, methods for generating said plants, and molecular markers corresponding to SEQ ID NO: 1 and SEQ ID NO: 2, cDNA sequences thereof, fragments of at least 20 consecutive nucleotides thereof, and complementary sequences thereof.

Said Calibrachoa plants comprising a double-flowering characteristic and a dwarf growth characteristic comprising at least one of the SNPs selected from the group consisting of (i) the G to C nucleotide substitution at position number 320 of SEQ ID NO: 1 and (ii) the A to C nucleotide substitution at position number 247 in SEQ ID NO: 1, and (iii) the G to C nucleotide substitution at position 43 of SEQ ID NO: 2 may not be exclusively the result of the sexual crossing of plants.

The described Calibrachoa plant may further comprise an additional trait in its genome, which has been introduced or modified by a step of a technical nature so that the introduction or modification of that trait is not exclusively the result of the mixing of the genes of the plants chosen for sexual crossing. Said step of a technical nature may be selected from the group consisting of spontaneous mutagenesis, induced random mutagenesis, and targeted mutagenesis.

Additional traits are well known to the person skilled in the art an may include one or more of the following:
- The radially symmetric colouration as described in US20200253142A1. The radially symmetric pattern is described to be the result of a mutation and controlled by a single half-dominant gene. Varieties carrying the mutation are described in the patent.
- Tobamovirus resistance as described in WO 2019/174721 A1. The resistance is based on a (T/C) point mutation in an endogenous gene which can be obtained by ems mutagenesis.
- The trait "Chamelon" trait which causes colour change during a growing season. The trait is caused by a dominant mutation. Varieties are available on the market.
- The "bicolour trait" which results in differently petaloide with a different colour in comparison to the main colour of the flower. Also this trait is caused by a recessive mutation. Varieties are publically available.

Said additional trait may be an additional mutation affecting flower colour or flower colour pattern, wherein said mutation may be the result of an induced random or targeted mutagenesis. Colour mutation of Calibrachoa are - for example disclosed in US 2017/0071117 (Calibrachoa Plant Named "uscalchstm'),

For said Calibrachoa plant, at least one of the SNPs selected from the group consisting of (i) the G to C nucleotide substitution at position number 320 of SEQ ID NO: 1 and (ii) the A to C nucleotide substitution at position number 247 in SEQ ID NO: 1, and (iii) the G to C nucleotide substitution at position 43 of SEQ ID NO: 2 or the additional trait may not be exclusively obtained by essentially biological processes.

### History of the double-flowering trait, US Patent No. 7,786,342

Double-flowering *Calibrachoa* varieties were generated by Applicant through an intensive breeding program which began with screening 17,500 first generation plants. A handful of sections from these F₁ plants exhibited some flowers having more than 5 petals, and were then the subject of extensive breeding which included, for example, intercrossing siblings or half-siblings, back-crossing, out-crossing (to increase diversity and circumvent inbreeding depression), and open pollinations through the third and higher generations. Since then a number of double-flowering varieties have been developed, encompassing a wide range of colours and patterns (see Table 1 below).

**Table 1 - Double-flowering Calibrachoa varieties**

| **Denomination** | **Trade name** | **Patent No. / PVP No.** |
|---|---|---|
| KLECA20428 | Superbells^{®} Double Amber | |
| KLECA20459 | Superbells^{®} Double Orange | |
| KLECA14275 | Superbells^{®} Double Orchid | |
| KLECA19067 | MiniFamous^{®} Neo Double OrangeTastic | |
| KLECA18085 | MiniFamous^{®} Uno Double PinkTastic^{®} | |
| KLECA07162 | MiniFamous^{®} Double Blue | PP20,201 |
| KLECA08164 | MiniFamous^{®} Double Pink Blush | |
| KLECA08182 | Compact MiniFamous^{®} Double Yellow | PP21,018 |
| KLECA09204 | Compact MiniFamous^{®} Double Lemon | PP23,191 |
| KLECA09207 | MiniFamous^{®} Double Hot Pink | |
| KLECA09208 | MiniFamous^{®} Neo Double Amethyst | PP22,600 |
| KLECA10220 | MiniFamous^{®} Double Pink | |
| KLECA11225 | MiniFamous^{®} Neo Double Yellow | |
| KLECA11226 | MiniFamous^{®} Double Nostalgia | |
| KLECA12231 | MiniFamous^{®} Double Blue | |
| KLECA12233 | MiniFamous^{®} Double Purple Red | |
| KLECA12234 | MiniFamous^{®} Neo Double Pink Vein | |
| KLECA13242 | MiniFamous^{®} Uno Double White | |
| KLECA13255 | MiniFamous^{®} Neo Double Dark Red | |
| KLECA13257 | Compact MiniFamous^{®} Double Red | |
| KLECA14261 | MiniFamous^{®} Double Orchid | |
| KLECA14264 | MiniFamous^{®} Uno Double Pink | |
| KLECA15269 | MiniFamous^{®} Neo Double Blue '15 | |
| KLECA14272 | Can-Can Rosies Blue | |
| KLECA14273 | Can-Can Rosies Dark Yellow | |
| KLECA14274 | Can-Can Rosies White | |
| KLECA14275 | Can-Can Rosies Pink Vein | |
| KLECA14276 | MiniFamous^{®} Double Purple | |
| KLECA14277 | Can-Can Rosies Magenta | |
| KLECA14283 | MiniFamous^{®} Double Apricot | |
| KLECA15290 | MiniFamous^{®} Neo Double Lemon '15 | |
| KLECA15332 | Compact MiniFamous^{®} Double Rose | |
| KLECA16356 | MiniFamous^{®} Uno Double PinkMania! | |
| KLECA16364 | MiniFamous^{®} Neo Double Light Blue | |
| KLECA19508 | MiniFamous^{®} Uno Double Blue | |
| KLECA20509 | MiniFamous^{®} Neo Double light violet 098 | |
| KLECA18510 | MiniFamous^{®} Uno Double LavTastic | |
| KLECA18511 | MiniFamous^{®} Uno Double White Pink Vein | |
| KLECA18514 | MiniFamous^{®} Uno Double Red | |
| USCAL81302 | Superbells^{®} Doublette Love Swept^{™} | PP30,804 |
| USCAL83901 | Superbells^{®} Double Ruby | Can4591 |
| US08CJ0202 | Superbells^{®} Double Rose | |
| USCAL51505 | Superbells^{®} Double Chiffon | PP30,803 |
| Duealdubcit | Aloha Double Citric | NL238194 |
| Duealdublav | Aloha Double Lavender | NL238053 |
| Dueldubstra | Aloha Double Strawberry | NL238195 |
| | Aloha Double Orange | |
| | Aloha Double Pink | |
| | Aloha Double Purple | |
| | Aloha Double Cherry Red | |
| DCALNOADBZ | Noa^{™} Double Bronze | |
| | Noa^{™} Double Pineapple | |
| WESCADOBL(U) | Celebration Double Blue | PP21,525 |
| WESCADODARE | Celebration Double Dark Red | |
| WESCADOFU | Celebration Double Fuchsia | |
| WESCADOLEM | Celebration Double Lemon | |
| WESCADOORE | Celebration Double Orange Pink | |
| WESCADOPI | Celebration Double Pink | PP21,465 |
| WESCADOSOPI | Celebration Double Soft Pink | |
| WESCADOWEIM | Celebration Double White Improved | |
| WESCADOYEL | Celebration Double Yellow | |
| WESCACHADOPIYE | CHAMELEON^{®} Double Pink Yellow | |
| WESCACHAMTIPI | CHAMELEON^{®} Double Ticld Pink | |
| | COLIBRI^{™} Double Copper | |

As with all *Calibrachoa* varieties, these plants show high vigour and therefore growth regulators need to be applied in order to achieve a more compact plant shape, which is commercially desired.

### History of the dwarf growth trait

*Calibrachoa* varieties naturally show high vigour and therefore plant growth regulators (PGR) need to be applied in order to achieve a more compact plant shape, which is commercially and economically desired. However, growth regulators are increasingly being banned. For instance, the growth regulator TILT^{®} is banned in the USA, Canada, Germany and Sweden. Additionally, the timing of PGR application is very important for the shape of end products, but the correct moment of application is influenced by temperature and development stages of the plants. This means that it is difficult for a grower to apply PGR at the correct moment. Incorrect PGR application regularly leads to economic loss during cultivation. It is therefore also commercially interesting to breed *Calibrachoa* plants which can be grown without PGRs.

*Calibrachoa* plants with dwarf phenotypes have been described and are known in the art (see Table 2a below, for example). These example varieties, while sometimes labelled "compact" are in fact distinct from other compact varieties and are herein referred to as dwarf varieties. The dwarf trait is recessive and breeding with the dwarf trait has been difficult, thus there are a limited number of commercially available lines. Examples of compact varieties are shown in Table 2b.

**Table 2a - Dwarf Varieties**

| **Denomination** | **Trade name** | **Patent No. / PVP No.** |
|---|---|---|
| | MiniFamous^{®} Piu Pink | |
| SAKCAL106 | Calipetite^{®} Blue | NL242570 |
| SAKCAL105 | Calipetite^{®} Red | PP24,381; NL242571 |
| SAKCAL104 | Calipetite^{®} Rose | NL242572 |
| SAKCAL108 | Calipetite^{®} White | NL242568 |
| SAKCAL107 | Calipetite^{®} Yellow | NL242569 |
| Balcongraniss | Conga^{™} Orange Kiss | PBR51567 |
| Balcongcink | Conga^{™} Pink | PBR51563 |
| Balcongite | Conga^{™} White | PBR51565 |
| | Conga^{™} Peach Kiss | |
| Wescaebreim | Early Bird^{™} Red | |
| Wescaebsu | Early Bird^{™} Sun | |
| | Pocket^{™} Lilac | |
| | Pocket^{™} Yellow | |

**Table 2b - Compact varieties**

| **Denomination** | **Trade name** | **Patent No. / PVP No.** |
|---|---|---|
| KLECA17002 | MiniFamous^{®} Piu White | |
| KLECA17038 | MiniFamous^{®} Piu Red | PP30,835 |
| KLECA17288 | MiniFamous^{®} Piu Light Blue | |
| KLECA17338 | MiniFamous^{®} Piu Yellow | |
| KLECA17340 | MiniFamous^{®} Piu Yellow+Red Veins | |
| KLECA17343 | Calita^{®} Scarlet Red Eye | |
| | MiniFamous^{®} Piu Orange | |
| | Calita^{®} Compact Lemon | |
| SAKCAL114 | Calipetite^{®} Pink Vein | NL243713 |
| SAKCAL110 | Calipetite^{®} Plum | NL242573 |
| Balcongetiss | Conga^{™} Sunset Kiss | |
| Balcongarlu | Conga^{™} Dark Blue | PP29,491; PBR51562 |
| Balcongcriss | Conga^{™} Coral Kiss | NL246124 |
| Balconglow | Conga^{™} Lemon | NL246125 |
| Balcongor | Conga^{™} Orange | NL246126 |
| Balconginkiss | Conga^{™} Pink Kiss | NL246123 |
| Balcongosiss | Conga^{™} Rose Kiss | NL246326 |
| Balcabscarim | Conga^{™} Red | |
| Balconglipar | Conga^{™} Light Pink Star | |
| Balconginar | Conga^{™} Pink Star | |
| Balconglav | Conga^{™} Lavender | PP30,114 |
| Balcongrissm | Conga^{™} Rose Kiss | PBR51568 |
| Balcongdel | Conga^{™} Deep Yellow | PBR51564 |
| Balcongosiss | Conga^{™} Rose | PBR51566 |
| Wescaebblim | Early Bird^{™} Blue | |
| Wescaebli | Early Bird^{™} Lilac | |
| Wescaebpi | Early Bird^{™} Pink | |
| Wescaebwe | Early Bird^{™} White | |
| | Pocket^{™} Apricot Eye | |
| | Pocket^{™} Dark Pink | |
| | Pocket^{™} Light Red | |
| | Pocket^{™} Rose | |
| | Pocket^{™} White | |
| | Aloha Nani Yellow | |
| | Aloha Nani Cherry Red | |
| | Aloha Nani Blue | |
| | Aloha Nani Dark Red | |
| | Aloha Nani Tropicana | |
| | Aloha Nani Red Cart Wheel | |
| | Aloha Nani White | |
| | Aloha Nani Golden Girl | |
| | Colibri^{™} Malibu Pink | |
| | Colibri^{™ Mellow} Yellow | |
| | Colibri^{™} Pink Flamingo | |
| | Colibri^{™} Yellow Canary | |
| DCALCOLBL | Colibri^{™} Blizzard | |
| | Colibri^{™} Lemon | |
| DCALCOPILA | Colibri^{™} Pink Lace | |
| DCALCOCHLA | Colibri^{™} Cherry Lace | |
| DCALCORANG | Colibri^{™} Orange | |
| | Colibri^{™} Pink | |
| DCALCOPULA | Colibri^{™} Purple Lace | |
| DCALCOFUCH | Colibri^{™} Fuchsia | |
| | Colibri^{™} Plum | |

Thus, in order for the double-flowering and dwarf traits to be combined, one would require the underlying molecular mechanisms and/or markers for any successful breeding program.

### Detection of single nucleotide polymorphisms (SNP) associated with the mitochondrial allele that causes a double-flowering phenotype

A diverse collection of *Calibrachoa* plants, were phenotypically and genetically analysed. The experimental trials for this analysis were grown in multi-location sites worldwide over several years. The plant cultivation tests were conducted in an experimental design containing several sub-experiments to capture the crop growth performance affected by different or no inhibiting substances (Paclobutrazol, Daminozide, no plant growth regulator). In total, 464 plants were analysed (343 single flowering and 121 double-flowering).

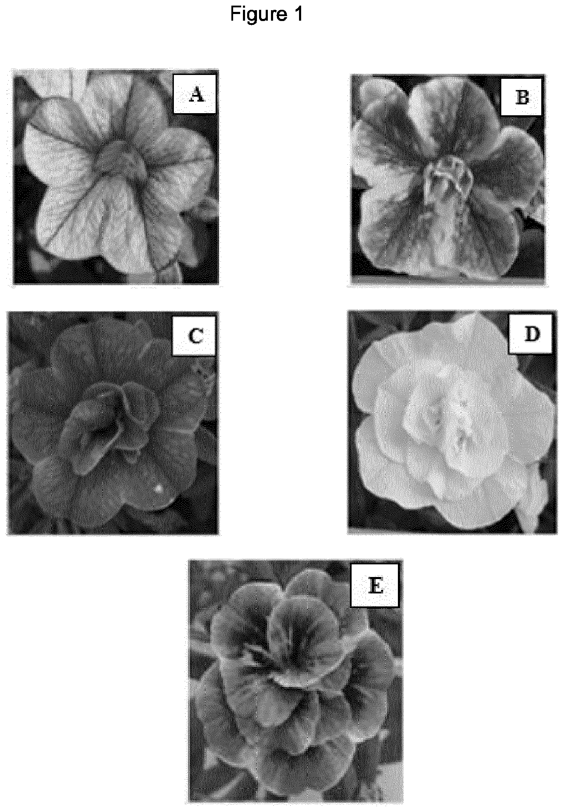

For the phenotypic analysis, the level of flower filling, or the petaloid stamina rating, was scored visually on a 1 - 9 scale, based on the UPOV scale for quantitative traits, where 1 means single flowers having five petals per flower and a score of 2 or higher means flowers containing converted anthers. See for example Figures 1A-1E and Table 3 below.

**Table 3 - States of Double Flower Expression**

| Approximate percentage of flower dimeter covered by additional petals or petaloids | Scale |
|---|---|
| >1 | 1 |
| 1 - 10 | 2 |
| 10.1 - 30 | 3 |
| 30.1 - 40 | 4 |
| 40.1 - 50 | 5 |
| 50.1 - 60 | 6 |
| 60.1 - 70 | 7 |
| 70.1 - 80 | 8 |
| > 80 | 9 |

As shown in Figures 1A-1E and described above in Table 3, a plant having a petaloid stamina rating of 1 exhibits single flowers with five petals (Figure 1A). Plants having a petaloid stamina rating of 2 have rudimentary converted anthers, while plants scoring 3 (Figure 1B) or higher covering a bigger size of the total flower diameter by additional petals or petaloids.

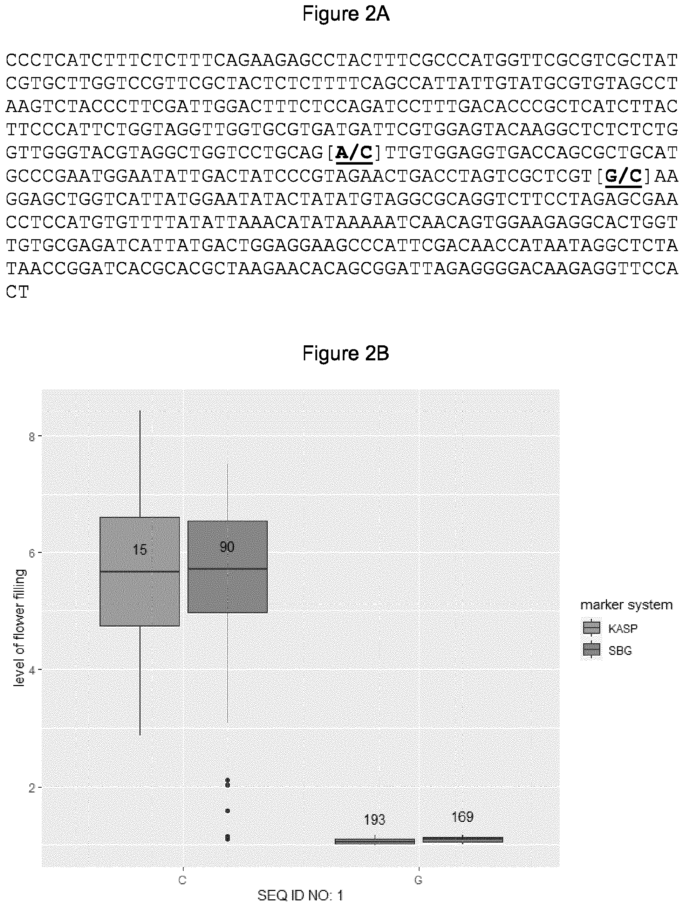

For the genotypic analysis, an applied Sequence Based Genotyping (SBG) approach for the simultaneous Single-Nucleotide Polymorphism (SNP) discovery was conducted to genotype 288 plants (186 double-flowering, 102 single flowering). SBG libraries were prepared on the basis of genomic Deoxyribonucleic Acid (DNA) including both nuclear and mitochondrial DNA of 288 *Calibrachoa* samples and subsequently sequenced using the Illumina HiSeq. The resulting alignments were subsequently mined for SNP mutations (Truong et al. 2012). Quality checks were performed, leading to 11,641 SNP mutation markers. A Genome-Wide Association Study (GWAS) implemented with the high-quality data revealed a major Quantitative Trait Locus (QTL) in which one of the alleles is responsible for double-flowering phenotypes. The nucleotides between brackets in Figure 2A indicate the position of the SNP mutations: an A to C nucleotide substitution at position number 247 in SEQ ID NO: 1 and a G to C nucleotide substitution at position number 320 of SEQ ID NO: 1. Genotypes having the "G" allele at position 320 displayed in all cases the single flower phenotypic trait. Genotypes having the "C" allele at position 320 displayed in all cases the double-flowering phenotype.

Other than for the SNP mutations described above, the sequence shown by SEQ ID NO: 1 is identical in the single and the double-flowering lines. The findings were confirmed with a Competitive Allele Specific PCR (KASP) assay (Chunlin et al. 2014). For the validation with the KASP, 234 plants were genotyped (210 single flowering, 24 double-flowering). 58 genotypes were analysed with both SBG and KASP parallel.

### Scoring and genotyping the double-flowering trait

The data from the SBG and KASP assay was further evaluated using linear mixed model analysis adjusted for influencing factors like genotype-environment-interactions (Best linear unbiased Predictors).

The results of the analysis are shown in Figure 2B, which depicts a boxplot of the level of flower filling (y-axis) of genotypes having a C at position number 320 of SEQ ID NO: 1 compared to those having a G at position 320 of SEQ ID NO: 1 (x-axis). The validation of the findings within the SBG approach (turquoise box plots, 259 of the 288 plants analysed) is displayed with additional box plots for the KASP assay (red box plots, 208 of the 234 plants analysed). The number within the boxplot indicates the number of genotypes in each group, while the outward lines indicate the minimum and maximum values. As shown in Figure 2B, plants having a C at position number 320 of SEQ ID NO: 1 have transformed anthers to additional petals or petaloids a flower filling rating of at least 2, while plants having a G at position 320 of SEQ ID NO: 1 exhibited a flowering filing rating of 1.

Thus, an embodiment of the present disclosure provides a molecular marker for distinguishing a plant having an allele for a double-flowering characteristic comprising at least one sequence selected from the group consisting of SEQ ID NO: 1, cDNA sequences thereof, fragments of at least 20 consecutive nucleotides thereof, and complementary sequences thereof. As will be understood by one skilled in the art, fragments may comprise lengths of at least 30, at least 40, at least 50, at least 60, least 70, at least 80, at least 90, at least 100, at least 125, at least 150, at least 175, at least 200, etc., nucleotides and upwards to the entire length of the sequence.

As will be understood by those skilled in the art, the SNP mutations shown in SEQ ID NO: 1 may be detected by any number of mechanisms, including but not limited to, Restriction Fragment Length Polymorphisms (RFLPs), Dynamic Allele-Specific Hybridization (DASH), molecular beacon, SNP microarray, PCR-based method, Flap endonuclease (FEN), Single-strand conformation polymorphism, temperature gradient gel electrophoresis, Denaturing High Performance Liquid Chromatography (DHPLC), DNA mismatch binding proteins, or sequencing.

Another embodiment of the present disclosure teaches a method for distinguishing a plant having at least one allele for a double-flowering characteristic comprising using SEQ ID NO: 1, and detecting at least one of a C nucleotide at position number 320 of SEQ ID NO: 1 and a C nucleotide at position number 247 in SEQ ID NO: 1. Detecting at least one of a C nucleotide at position number 320 of SEQ ID NO: 1 and a C nucleotide at position number 247 in SEQ ID NO: 1 may comprise obtaining genetic material, obtaining a nucleic acid, wherein said nucleic acid has at least a portion of sequence complementary to the molecular marker for the double-flowering trait disclosed herein, and base-pairing said nucleic acid with said genetic material and examining the result of said base-pairing. The genetic material may be deoxyribonucleic acid, ribonucleic acid, or a combination thereof. The nucleic acid may be a primer set, a probe, or combination thereof.

Further described are double-flowering dwarf *Calibrachoa* plants having a petaloid stamina rating of at least 2. Also described are double-flowering dwarf *Calibrachoa* plants exhibiting male sterility.

### Detection of single nucleotide polymorphisms (SNP) associated with the dwarf phenotype

The diverse collection of *Calibrachoa* plants described above (464 plants total), including a wide range of growth vigour from very dwarf to very vigorous varieties were phenotypically and genetically analysed. The experimental trials were grown in multi-location sites worldwide over several years. The plant cultivation tests were conducted in an experimental design containing several sub-experiments to capture the crop growth performance affected by different or no inhibiting substances (Paclobutrazol, Daminozide, no plant growth regulator).

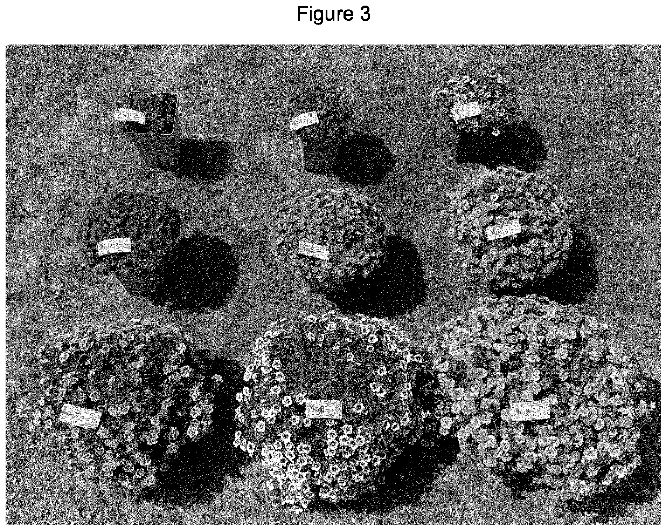

For the phenotypic analysis, growth vigour, or the determination of biomass, was rated on a 1 - 9 scale, where 1 refers to very dwarf and 9 to extreme vigorous plants. Plants representing each rating are shown in Figure 3, specifically, in the foreground, from left to right, are plants exhibiting ratings of 7, 8, and 9, respectively. The middle row, from left to right, shows plants exhibiting ratings of 4, 5, and 6, respectively. In the background, from left to right, are plants exhibiting ratings of 1, 2, and 3, respectively.

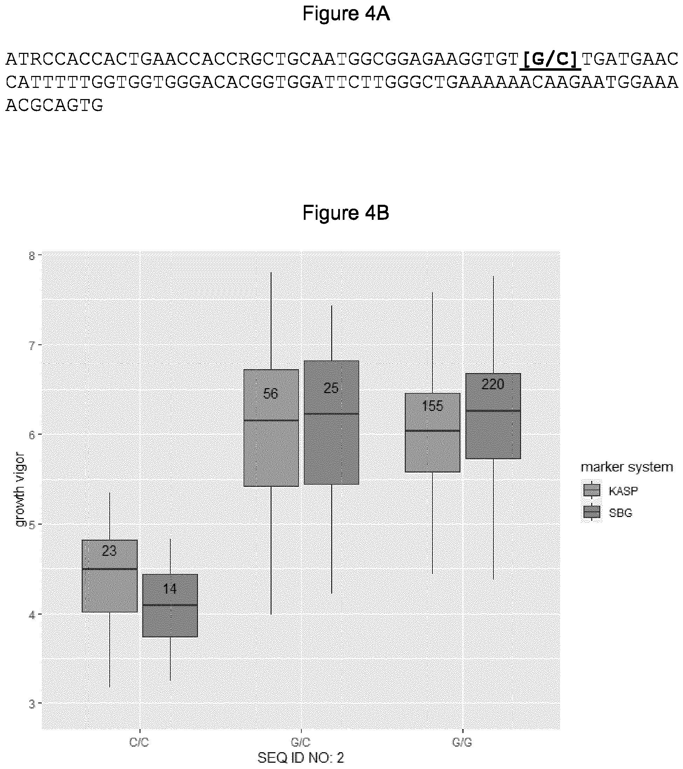

The genotypic analysis described above further revealed a major Quantitative Trait Locus (QTL) in which one of the alleles is responsible for a dwarf phenotype. The nucleotides between brackets in Figure 4A indicate the position of the SNP mutation comprising a G to C nucleotide substitution at position number 43 of SEQ ID NO: 2. Genotypes heterozygous or homozygous for the "G" allele at position 43 displayed in all cases the normal, vigorous plant growth. Genotypes homozygous for the "C" allele at position 43 displayed in all cases the dwarf phenotype. The confirmation of these findings was conducted with a Competitive Allele Specific PCR (KASP) assay.

### Scoring and genotyping the dwarf trait

The data from the SBG and KASP assay was further evaluated using linear mixed model analysis adjusted for influencing factors like genotype-environment-interactions (Best linear unbiased Predictors).

The results of the analysis are shown in Figure 4B, which depicts a boxplot of the level of growth vigour (y-axis) of genotypes homozygous for C at position number 43 of SEQ ID NO: 2 compared to those heterozygous or homozygous for G at position 43 of SEQ ID NO: 2 (x-axis). The validation of the findings within the SBG approach (turquoise box plots, 259 of the 288 plants analysed) is displayed with additional box plots for the KASP assay (red box plots, 234 plants analysed). The number within the boxplot indicates the number of genotypes in each group; while the outward lines indicate the minimum and maximum values. As shown in Figure 4B, plants homozygous for C at position number 43 of SEQ ID NO: 2 exhibited much less growth vigour, while plants having at least one G allele at position 43 of SEQ ID NO: 2 exhibited a significantly stronger growth vigour.

Thus, an embodiment of the present disclosure provides a molecular marker for distinguishing a plant having at least one allele for a dwarf characteristic comprising at least one sequence selected from the group consisting of SEQ ID NO: 2, cDNA sequences thereof, fragments of at least 20 consecutive nucleotides thereof, and complementary sequences thereof. As will be understood by one skilled in the art, fragments may comprise lengths of at least 30, at least 40, at least 50, at least 60, least 70, at least 80, at least 90, at least 100, at least 125, at least 150, at least 175, at least 200, etc., nucleotides and upwards to the entire length of the sequence.

As will be understood by those skilled in the art, the SNP mutation shown in SEQ ID NO: 2 may be detected by any number of mechanisms, including but not limited to, Restriction Fragment Length Polymorphisms (RFLPs), Dynamic Allele-Specific Hybridization (DASH), molecular beacon, SNP microarray, PCR-based method, Flap endonuclease (FEN), Single-strand conformation polymorphism, temperature gradient gel electrophoresis, Denaturing High Performance Liquid Chromatography (DHPLC), DNA mismatch binding proteins, or sequencing.

Another embodiment of the present disclosure teaches a method for distinguishing a plant having at least one allele for a dwarf characteristic comprising using SEQ ID NO: 2, and detecting a C nucleotide at position 43 of SEQ ID NO: 2. Detecting a C nucleotide at position 43 of SEQ ID NO: 2 may e.g. comprise obtaining genetic material, obtaining a nucleic acid, wherein said nucleic acid has at least a portion of sequence complementary to the molecular marker for the dwarf trait disclosed herein, and base-pairing said nucleic acid with said genetic material and examining the result of said base-pairing. The genetic material may be deoxyribonucleic acid, ribonucleic acid, or a combination thereof. The nucleic acid may be a primer set, a probe, or combination thereof.

Also described is a plant distinguished by the markers and methods disclosed herein, wherein said plant is homozygous for said allele for a dwarf growth characteristic, and wherein said plant is subsequently grown without growth regulators. The plant may comprise no detectable residue of a synthetic plant growth regulator or a related breakdown of a plant growth regulator product.

Also described are double-flowering dwarf *Calibrachoa* plants having a significantly smaller growth vigour rating of less than 5 at maturity, when compared to plants having a G/C or G/G genotypes at position 43 of SEQ ID NO: 2 when grown under the same environmental conditions.

### Plant growth regulators (PGRs)

Plant growth regulators (PGRs) (herein also called synthetic plant growth regulators) are widely used in the ornamental plant business. PGRs consist of a large group of synthetically produced organic chemicals and considered as helping tool in the actual production system of ornamentals. The application of them is exercised by the commercial growers as a part of cultural practice. There are many methods of application of PGRs, most common used is drenching, foliar spraying and pre-plant soaking. According to professional experience and literature research, one or more PGRs have been used in *Calibrachoa* cultivation (Table 4 below).

**Table 4: Widely used Plant Growth Regulators (PGRs) for Calibrachoa (modified according Wipker 2013 and 2019)**

| **PGR** | **Active Ingredient** | **Application rate** | **Method** |
|---|---|---|---|
| Dazide (B-Nine) | Daminozide | 2,500 - 5,000 ppm | Spray |
| Citadel (Cycocel) + Dazide (B-Nine) | Chlormequat + Daminozide | 1,500 ppm (Citadel) + 2,500 ppm (Dazide) | Tank-mix spray |
| Concise (Sumagic) | Uniconazole-p | 10 - 25 ppm | Spray |
| Piccolo (Bonzi, Paczol, Downsize)) | Paclobutrazol | 3 - 8 ppm | Drench |
| Piccolo (Bonzi, Paczol) | Paclobutrazol | 3 - 50 ppm | Spray |
| Florel | Ethephon | 300 - 500 ppm | Spray |
| Toplor | Flurprimidol | 150 - 300 ppm | Spray |

The dangerous effects on both grower and end consumer health have increasingly become the focus of awareness worldwide. Various countries and international organizations have issued regulations by setting up the maximum authorized residue levels of PGRs in various plants and this will likely intensify in the future.

A large number of analytical methods for the determination of PGRs or residues of them have been developed. The methods of detection are enzyme-linked immunosorbent assay (ELISA) (Qian et al. 2009; Jiang et al. 2011), gas chromatography (GC) (Brinkmann et al. 1996; Xu et al. 2011), liquid chromatography-diode array detection (LC-DAD) (Das and Prasad 2015), gas chromatography-mass spectrometry (GC-MS) (Müller et al. 2002; Du et al. 2015), liquid chromatography-tandem mass spectrometry (LC-MS/MS) (Riediker et al. 2002; Ma et al. 2013; Kim et al. 2016), and high-performance liquid chromatography coupled with tandem mass spectrometry (HPLC-MS/MS) (Luo et al. 2019). Accredited laboratories (e.g. Analytisches Insitut Bostel, https://bostel.de/) analyse plant material for residue level conform to legal requirements (e.g. German Federal Office of Consumer Protection and Food Safety, § 64 LFGB).

The double-flowering dwarf *Calibrachoa* plants of the present disclosure inherently exhibit a significantly smaller growth vigour. The present disclosure also provides for double-flowering dwarf *Calibrachoa* plants grown without the addition of synthetic plant growth regulators. Figure 4C shows three *Calibrachoa* plants of two different genotypes. *Calibrachoa* plant I is a genotype with a combination of the double-flowering trait (SEQ ID NO: 1; "C") and a dwarf growth trait (SEQ ID NO: 2; "C/C"), not treated with any plant growth regulators and has a vigour rating of 3. *Calibrachoa* plants II and III demonstrate the same genotype with the double-flowering trait (SEQ ID NO: 1; "C") but do not comprise the dwarf growth trait (SEQ ID NO: 2; "G/G"). Plant II is untreated with any plant growth regulators, and has a vigour rating of 7. Plant III was growth inhibited according to good horticultural practice (5 treatments of Dazide/B-Nine) and has a vigour rating of 6.

Also described are provides for double-flowering dwarf *Calibrachoa* plants which comprise no detectable residue of a synthetic plant growth regulator or a related breakdown of a plant growth regulator product.

### Methods of a producing double-flowering dwarf Calibrachoa

Described is a method of producing a *Calibrachoa* plant comprising a double-flowering characteristic and a dwarf growth characteristic comprising the steps of: crossing a first female *Calibrachoa* plant with a first male *Calibrachoa* plant to produce F₁ plants, wherein said first female *Calibrachoa* plant comprises a mitochondrial allele associated with at least one SNP mutation selected from the group consisting of (i) a G to C nucleotide substitution at position number 320 of SEQ ID NO: 1 and (ii) an A to C nucleotide substitution at position number 247 in SEQ ID NO: 1 and exhibiting a double-flowering characteristic, and wherein said first male *Calibrachoa* plant has at least one copy of a nuclear, recessive allele associated with a SNP mutation consisting of a G to C nucleotide substitution at position 43 of SEQ ID NO: 2, wherein when said nuclear allele is in the homozygous form plants exhibit a dwarf growth characteristic; screening said F₁ plants for the presence of said nuclear SNP mutation; selecting an F₁ female plant exhibiting said double-flowering characteristic and further comprising at least one copy of said nuclear SNP mutation; crossing said F₁ female plant with said first male or a second male *Calibrachoa* plant having at least one copy said nuclear SNP mutation to produce F₂ plants; screening said F₂ plants for the presence of said nuclear SNP mutation; and selecting an F₂ plant exhibiting said double-flowering characteristic and being homozygous for said nuclear SNP mutation. The first or second male *Calibrachoa* plant can e.g.be homozygous for said nuclear SNP mutation and exhibits a dwarf growth characteristic.

Said crossing with said first male Calibrachoa plant can introduce at least one copy of a nuclear, recessive allele associated with a SNP mutation consisting of a G to C nucleotide substitution at position 43 of SEQ ID NO: 2 into the genome of the resulting Calibrachoa plant.

Further described is a method of selecting a Calibrachoa plant comprising a double-flowering characteristic and a dwarf growth characteristic, comprising the steps of: crossing a first female Calibrachoa plant with a first male Calibrachoa plant to produce F1 plants, wherein said first female Calibrachoa plant comprises a mitochondrial allele associated with at least one SNP mutation selected from the group consisting of (i) a G to C nucleotide substitution at position number 320 of SEQ ID NO: 1 and (ii) an A to C nucleotide substitution at position number 247 in SEQ ID NO: 1 and exhibiting a double-flowering characteristic, and wherein said first male Calibrachoa plant has at least one copy of a nuclear, recessive allele associated with a SNP mutation consisting of a G to C nucleotide substitution at position 43 of SEQ ID NO: 2, wherein, when said nuclear allele is in the homozygous form plants exhibit a dwarf growth characteristic; screening said F1 plants for the presence of said nuclear SNP mutation; selecting an F1 female plant exhibiting said double-flowering characteristic and further comprising at least one copy of said nuclear SNP mutation; crossing said F1 female plant with said first male or a second male Calibrachoa plant having at least one copy of said nuclear SNP mutation to produce F2 plants; screening said F2 plants for the presence of said nuclear SNP mutation; and selecting an F2 plant exhibiting said double-flowering characteristic and being homozygous for said nuclear SNP mutation.

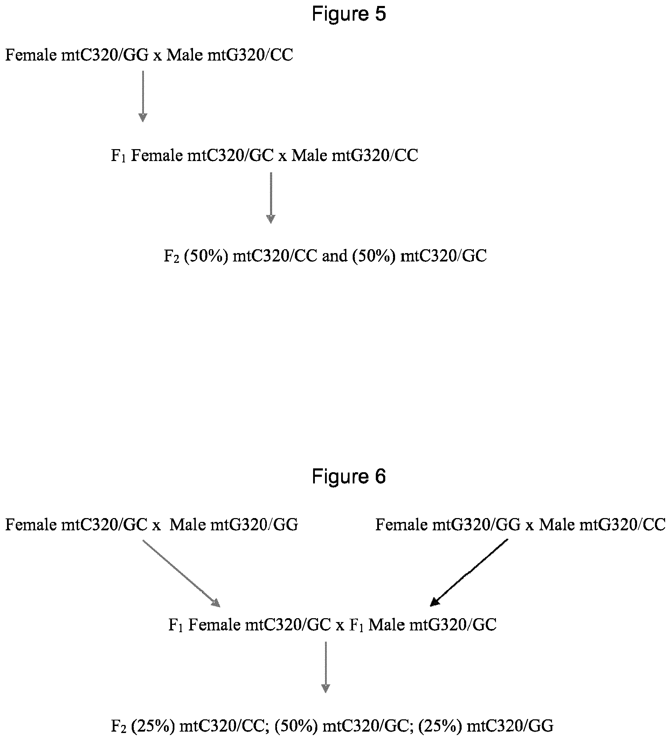

As will be understood by those skilled in the art, the first and/or second male *Calibrachoa* plant may be homozygous for the nuclear dwarf growth characteristic. As shown in Figure 5, a first female *Calibrachoa* plant comprising a mitochondrial allele associated with a G to C nucleotide substitution at position number 320 of SEQ ID NO: 1 and (ii) an A to C nucleotide substitution at position number 247 in SEQ ID NO: 1 and exhibiting a double-flowering characteristic (mtC320) with vigorous growth G/G) is crossed with a male exhibiting single flowers (mtG320) and a dwarf growth trait (C/C). An F₁ female progeny from this cross having a double-flowering characteristic (mtC320) and vigorous growth (G/C) is then bred to a second male exhibiting single flowers (mtG320) and a dwarf growth trait (C/C). While Figure 5 depicts this as an outcrossing, those skilled in the art will understand that this may also be accomplished via backcrossing to the first male *Calibrachoa.* 50% of the F₂ progeny from this cross will exhibit double-flowering and dwarf growth, and the other 50% will exhibit double-flowering and normal, vigorous growth as they will be heterozygous for the dwarf allele (G/C).

As will be understood by those skilled in the art, additional crosses may be conducted to produce the parental lines described above. Shown in Figure 6 is an example wherein a double-flowering female (mtC320) carrying the dwarf allele (G/C) is crossed with a male having single flowers (mtG320) and no dwarf allele (G/G) but may comprise some other desirable trait (not depicted). An F₁ progeny is selected for having double-flowers (mtC320) and carrying the dwarf allele (G/C) and additional desired trait (not depicted) to be used as the female parent in a cross with a male having single flowers (mtG320) and carrying the dwarf allele (G/C). This male parental line may have been produced to carry, for example, the same desirable trait as the F₁ female parental line, or may carry a second desirable trait, and may have been produced as depicted in Figure 6, from a cross between a single-flowering (mtG320) vigorous female (G/G) with a single flowering (mtCG20) dwarf male (C/C). As will be understood by those skilled the art, the F₁ male parental line carrying the dwarf allele shown in Figure 6 may have been produced by any number of crosses, for example, by one or both parental lines being heterozygous for the dwarf allele.

As shown in Figure 6, all F₂ progeny will exhibit double-flowering (mtC320) as this is inherited from the mitochondria of the F₁ female parent. Additionally, approximately 25% of F₂ progeny will also be homozygous for the dwarf allele and exhibit the dwarf growth trait (C/C).

### Additional Breeding Methods

Plants can be produced using the double-flowering dwarf plants and/or markers of the double-flowering and dwarf traits disclosed herein. These methods are well-known in the art and some of the more commonly used breeding methods are described herein. Descriptions of breeding methods can be found in one of several reference books (e.g., Allard, "Principles of Plant Breeding" (1999); and Vainstein, "Breeding for Ornamentals: Classical and Molecular Approaches," Kluwer Academic Publishers (2002); Callaway, "Breeding Ornamental Plants," Timber Press (2000).

Breeding steps that may be used in a plant breeding program can include for example, pedigree breeding, backcrossing, mutation breeding, and recurrent selection. In conjunction with these steps, techniques such as RFLP-enhanced selection, genetic marker enhanced selection (for example, SSR markers), gene editing and the making of double haploids may be utilized.

Described herein is a method of producing a double-flowering dwarf *Calibrachoa* plant having a desired trait comprising applying a plant breeding technique to the double-flowering dwarf *Calibrachoa* plant produced from the breeding methods disclosed herein. The desirable trait may be for example, a mutation affecting flower colour and/or pattern. Thus, the present disclosure provides for plants produced by the breeding methods disclosed herein and further comprising a mutation affecting flower colour and/or pattern.

The present disclosure further provides for plants produced by the breeding methods disclosed herein, wherein said plant has a petaloid stamina rating of at least three and wherein said plant at maturity has a vigour rating of less than five compared to plants having a G/C or G/G genotype at position 43 of SEQ ID NO: 2 when grown under the same environmental conditions.

Plants produced from the breeding methods disclosed herein may be asexually propagated or sexually reproduced. The plants produced can be grown without plant growth regulators.

### Recurrent Selection and Mass Selection

The plant breeding technique can be recurrent selection or mass selection. Mass and recurrent selections can be used to improve populations of either self- or cross-pollinating crops. A genetically variable population of heterozygous individuals is either identified, or created, by intercrossing several different parents. The plants are selected based on individual superiority, outstanding progeny, or excellent combining ability. The selected plants are intercrossed to produce a new population in which further cycles of selection are continued.

Recurrent selection is a method used in a plant breeding program to improve a population of plants. Double-flowering dwarf *Calibrachoa* plants disclosed herein are suitable for use in a modified recurrent selection program. The method entails individual plants cross pollinating with each other to form progeny. The progeny are grown and the superior progeny selected. The selected progeny are cross pollinated with each other to form progeny for another population. This population is planted and again superior plants are selected to cross pollinate with each other. Recurrent selection is a cyclical process and therefore can be repeated as many times as desired. The objective of recurrent selection is to improve the traits of a population. The improved population can then be used as a source of breeding material to obtain new varieties for commercial or breeding use, including the production of a synthetic variety. A synthetic variety is the resultant progeny formed by the intercrossing of several selected varieties. Once a desired plant is obtained with improved traits, it can be used as a breeding line in crosses to generate double-flowering dwarf *Calibrachoa* plants.

Mass selection is a useful technique when used in conjunction with molecular marker enhanced selection. In mass selection, seeds from individuals are selected based on phenotype or genotype. These selected seeds are then bulked and used to grow the next generation. Bulk selection requires growing a population of plants in a bulk plot, allowing the plants to self-pollinate, harvesting the seed in bulk, and then using a sample of the seed harvested in bulk to plant the next generation. In addition to self-pollination, directed pollination could be used as part of the breeding program.

### Open-pollination and hybridization

The plant breeding technique can be open-pollination. Open-pollination is when pollination occurs by insect, bird, wind, humans, or other natural mechanisms. This can yield greater variation and more genetically diverse plants. The plant breeding technique can be hybridization. Hybridization is a controlled method of pollination in which the pollen of two different varieties or species is crossed by human intervention. For example, *Calibrachoa* can hybridize with petunia to produce *petunia-Calibrachoa* hybrids. Thus, also disclosed are *petunia-Calibrachoa* hybrids exhibiting the double-flowering and/or dwarf traits disclosed herein.

### Backcross breeding

The plant breeding technique can e.g. be backcrossing. Backcross breeding has been used to transfer genes for a simply inherited, highly heritable trait into a desirable homozygous variety or inbred variety which is the recurrent parent. The source of the trait to be transferred is called the donor parent. After the initial cross, individuals possessing the phenotype of the donor parent are selected and repeatedly crossed (backcrossed) to the recurrent parent. The resulting plant is expected to have the attributes of the recurrent parent (e.g., variety) and the desirable trait transferred from the donor parent. This is also known as single gene conversion and/or backcross conversion.

In addition to being used to create a backcross conversion, backcrossing can also be used in combination with pedigree breeding. Backcrossing can be used to transfer one or more specifically desirable traits from one variety, the donor parent, to a developed variety called the recurrent parent, which has overall good commercial characteristics yet lacks that desirable trait or traits. However, the same procedure can be used to move the progeny toward the genotype of the recurrent parent, but at the same time retain many components of the nonrecurrent parent by stopping the backcrossing at an early stage and proceeding with selection. This approach leverages the value and strengths of the recurrent parent for use in new *Calibrachoa* varieties.

### Pedigree Breeding

The plant breeding technique can e.g. be pedigree breeding. Pedigree breeding starts with the crossing of two genotypes, such as the double-flowering and dwarf alleles disclosed herein, and another variety having one or more desirable characteristics that is lacking or which complements double-flowering dwarf *Calibrachoa* plants. If the two original parents do not provide all the desired characteristics, other sources can be included in the breeding population. In the pedigree method, superior plants are selected in successive filial generations.

### Mutation breeding

The plant breeding technique can e.g. be mutation breeding, wherein said mutation breeding selects for a mutation that is spontaneous or artificially induced. Mutation breeding is another method of introducing new traits into the double-flowering dwarf *Calibrachoa* plants of the present disclosure. Mutations that occur spontaneously or are artificially induced can be useful sources of variability for a plant breeder. The goal of artificial mutagenesis is to increase the rate of mutation for a desired characteristic. Mutation rates can be increased by many different means. Examples of mutagens that may be used with the method disclosed herein include: radiation; such as X-rays, Gamma rays (e.g., cobalt 60 or caesium 137), neutrons, (product of nuclear fission by uranium 235 in an atomic reactor), Beta radiation (emitted from radioisotopes such as phosphorus 32 or carbon 14), or ultraviolet radiation (for example from 250 to 290 nm), temperature, long-term seed storage, tissue culture conditions, or chemical mutagens (such as base analogues (5-bromo-uracil)), related compounds (8-ethoxy caffeine), antibiotics (streptonigrin), alkylating agents (sulphur mustards, nitrogen mustards, epoxides, ethyleneamines, sulphates, sulfonates, sulfones, lactones), azide, hydroxylamine, nitrous acid, or acridines, proflavine, ICR191 and ethidium bromide. Other techniques such as gene editing are also possible and lie well within the scope of the skilled person.

Once a desired trait is observed through mutagenesis the trait may then be incorporated into existing germplasm by traditional breeding techniques. Details of mutation breeding can be found in Allard, Principles of Plant Breeding, John Wiley & Sons, Inc. (1960) but may include, for example, crossing, recurrent selection, backcrossing, pedigree breeding, marker enhanced selection, haploid/double haploid production, or transformation.

The double-flowering dwarf *Calibrachoa* plants as described herein may further comprise a mutation affecting flower colour and/or flower colour pattern, wherein said mutation is the result of a gene editing tool or technology.

### Breeding with Molecular Markers

A particular plant breeding technique described herein is marker enhanced selection. In addition to the sequences disclosed herein which may be used as molecular markers for the double-flowering and dwarf traits, molecular markers can be used during the breeding process for the selection of other traits. For example, markers closely linked to alleles or markers containing sequences within the actual alleles of interest can be used to select plants that contain the alleles of interest during a breeding program. The markers can also be used to select for the genome of the recurrent parent and against the genome of the donor parent. Using this procedure can minimize the amount of genome from the donor parent that remains in the selected plants. It can also be used to reduce the number of crosses back to the recurrent parent needed in a backcrossing program. The use of molecular markers in the selection process is often called genetic marker enhanced selection. Molecular markers may also be used to identify and exclude certain sources of germplasm as parental varieties or ancestors of a plant by providing a means of tracking genetic profiles through crosses.

Molecular markers, which includes markers identified through the use of techniques such as Isozyme Electrophoresis, Restriction Fragment Length Polymorphisms (RFLPs), Randomly Amplified Polymorphic DNAs (RAPDs), Arbitrarily Primed Polymerase Chain Reaction (AP-PCR), DNA Amplification Fingerprinting (DAF), Sequence Characterized Amplified Regions (SCARs), Amplified Fragment Length Polymorphisms (AFLPs), Simple Sequence Repeats (SSRs), and Single Nucleotide Polymorphisms, may be used in plant breeding methods. See Vainstein, "Breeding for Ornamentals: Classical and Molecular Approaches," Kluwer Academic Publishers (2002).

One use of molecular markers is Quantitative Trait Loci (QTL) mapping. QTL mapping is the use of markers, which are known to be closely linked to alleles that have measurable effects on a quantitative trait. Selection in the breeding process is based upon the accumulation of markers linked to the positive effecting alleles and/or the elimination of the markers linked to the negative effecting alleles from the plant's genome. See for example, Fletcher, Richard S., et al., "QTL analysis of root morphology, flowering time, and yield reveals trade-offs in response to drought in Brassica napus" Journal of Experimental Biology. 66 (1): 245-256 (2014). QTL markers can also be used during the breeding process for the selection of qualitative traits. For example, markers closely linked to alleles or markers containing sequences within the actual alleles of interest can be used to select plants that contain the alleles of interest during a backcrossing breeding program. The markers can also be used to select for the genome of the recurrent parent and against the genome of the donor parent. Using this procedure can minimize the amount of genome from the donor parent that remains in the selected plants. It can also be used to reduce the number of crosses back to the recurrent parent needed in a backcrossing program. The use of molecular markers in the selection process is often called genetic marker enhanced selection. Molecular markers may also be used to identify and exclude certain sources of germplasm as parental varieties or ancestors of a plant by providing a means of tracking genetic profiles through crosses.

### Molecular techniques using double-flowering dwarf Calibrachoa plants

The advent of new molecular biological techniques has allowed the isolation and characterization of genetic elements with specific functions. Traditional plant breeding has principally been the source of new germplasm, however, advances in molecular technologies have allowed breeders to provide varieties with novel and much wanted commercial attributes. Molecular techniques such as transformation are popular in breeding ornamental plants and well-known in the art. See Vainstein, "Breeding for Ornamentals: Classical and Molecular Approaches," Kluwer Academic Publishers (2002). Expression vectors can be introduced into plant tissues using a direct gene transfer method, such as microprojectile-mediated delivery, DNA injection, electroporation, and the like, or by using either microprojectile-mediated delivery with a biolistic device or by using Agrobacterium-mediated transformation.

### Expression vectors for Calibrachoa transformation: Marker genes

Plant transformation involves the construction of an expression vector which will function in plant cells. Such a vector comprises DNA comprising a gene under control of, or operatively linked to, a regulatory element (for example, a promoter). Expression vectors include at least one genetic marker operably linked to a regulatory element (for example, a promoter) that allows transformed cells containing the marker to be either recovered by negative selection, i.e., inhibiting growth of cells that do not contain the selectable marker gene, or by positive selection, i.e., screening for the product encoded by the genetic marker. Many commonly used selectable marker genes for plant transformation are well-known in the transformation arts, and include, for example, genes that code for enzymes that metabolically detoxify a selective chemical agent which may be an antibiotic or an herbicide, or genes that encode an altered target which is insensitive to the inhibitor. Positive selection methods are also known in the art.

One commonly used selectable marker gene for plant transformation is *neomycin phosphotransferase II* (nptll) which, when under the control of plant regulatory signals, confers resistance to kanamycin. Another commonly used selectable marker gene is the hygromycin phosphotransferase gene which confers resistance to the antibiotic hygromycin.

Selectable marker genes for plant transformation not of bacterial origin include, for example, mouse *dihydrofolate reductase,* plant *5-enolpyruvylshikimate-3-phosphate synthase,* and plant *acetolactate synthase* (Eichholtz, et al., Somatic Cell Mol. Genet., 13:67 (1987); Shah, et al., Science, 233:478 (1986); Charest, et al., Plant Cell Rep., 8:643 (1990)).

Another class of marker genes for plant transformation requires screening of presumptively transformed plant cells, rather than direct genetic selection of transformed cells, for resistance to a toxic substance such as an antibiotic. These genes are particularly useful to quantify or visualize the spatial pattern of expression of a gene in specific tissues and are frequently referred to as reporter genes because they can be fused to a gene or gene regulatory sequence for the investigation of gene expression. Commonly used marker genes for screening presumptively transformed cells include *β-glucuronidase* (GUS), *β-galactosidase, luciferase,* and *chloramphenicol acetyltransferase* (Jefferson, R. A., Plant Mol. Biol. Rep., 5:387 (1987); Teeri, et al., EMBO J., 8:343 (1989); Koncz, et al., Proc. Natl. Acad. Sci. USA, 84:131 (1987); DeBlock, et al., EMBO J., 3:1681 (1984)).

### Expression vectors for Calibrachoa transformation: Promoters

Genes included in expression vectors must be driven by a nucleotide sequence comprising a regulatory element (for example, a promoter). Several types of promoters are well known in the transformation arts as are other regulatory elements that can be used alone or in combination with promoters.

As used herein, "promoter" includes reference to a region of DNA upstream from the start of transcription and involved in recognition and binding of RNA polymerase and other proteins to initiate transcription. A "plant promoter" is a promoter capable of initiating transcription in plant cells. Examples of promoters under developmental control include promoters that preferentially initiate transcription in certain tissues, such as leaves, roots, seeds, fibres, xylem vessels, tracheids, or sclerenchyma. Such promoters are referred to as "tissue-preferred." Promoters that initiate transcription only in a certain tissue are referred to as "tissue-specific." A "cell-type" specific promoter primarily drives expression in certain cell types in one or more organs, for example, vascular cells in roots or leaves. An "inducible" promoter is a promoter which is under environmental control. Examples of environmental conditions that may affect transcription by inducible promoters include anaerobic conditions or the presence of light. Tissue-specific, tissue-preferred, cell-type specific, and inducible promoters constitute the class of "non-constitutive" promoters. A "constitutive" promoter is a promoter that is active under most environmental conditions. Many types of promoters are well known in the art.

### Signal sequences for targeting proteins to subcellular compartments

Transport of a protein produced by transgenes to a subcellular compartment, such as the chloroplast, vacuole, peroxisome, glyoxysome, cell wall, or mitochondrion, or for secretion into the apoplast, is accomplished by means of operably linking the nucleotide sequence encoding a signal sequence to the 5' and/or 3' region of a gene encoding the protein of interest. Targeting sequences at the 5' and/or 3' end of the structural gene may determine during protein synthesis and processing where the encoded protein is ultimately compartmentalized. Many signal sequences are well-known in the art. See, for example, Becker, et al., Plant Mol. Biol., 20:49 (1992); Knox, C., et al., Plant Mol. Biol., 9:3-17 (1987); Lerner, et al., Plant Physiol., 91 :124-129 (1989); Frontes, et al., Plant Cell, 3:483-496 (1991); Matsuoka, et al., Proc. Natl. Acad. Sci., 88:834 (1991); Gould, et al., J. Cell. Biol., 108:1657 (1989); Creissen, et al., Plant J., 2:129 (1991); Kalderon, et al., Cell, 39:499-509 (1984); Steifel, et al., Plant Cell, 2:785-793 (1990).

### Foreign genes: Transformation

Various promoters, targeting sequences, enhancing sequences, and other DNA sequences can be inserted into the genome for the purpose of altering the expression of genes.

Many techniques for altering gene expression are well-known to one of skill in the art, including, but not limited to, knock-outs (such as by insertion of a transposable element such as Mu (Vicki Chandler, The Maize Handbook, Ch. 118 (Springer-Verlag 1994)) or other genetic elements such as a FRT, Lox, or other site specific integration sites; antisense technology (see, e.g., Sheehy, et al., PNAS USA, 85:8805-8809 (1988) and U.S. Pat. Nos. 5,107,065, 5,453,566, and 5,759,829); co-suppression (e.g., Taylor, Plant Cell, 9:1245 (1997); Jorgensen, Trends Biotech., 8(12):340-344 (1990); Flavell, PNAS USA, 91:3490-3496 (1994); Finnegan, et al., BiolTechnology, 12:883-888 (1994); Neuhuber, et al., Mol. Gen. Genet., 244:230-241 (1994)); RNA interference (Napoli, et al., Plant Cell, 2:279-289 (1990); U.S. Pat. No. 5,034,323; Sharp, Genes Dev., 13:139-141 (1999); Zamore, et al., Cell, 101:25-33 (2000); Montgomery, et al., PNAS USA, 95:15502-15507 (1998)), virus-induced gene silencing (Burton, et al., Plant Cell, 12:691-705 (2000); Baulcombe, Curr. Op. Plant Bio., 2:109-113 (1999)); target-RNA-specific ribozymes (Haseloff, et al., Nature, 334:585-591 (1988)); hairpin structures (Smith, et al., Nature, 407:319-320 (2000); U.S. Pat. Nos. 6,423,885, 7,138,565, 6,753,139, and 7,713,715); MicroRNA (Aukerman & Sakai, Plant Cell, 15:2730-2741 (2003)); ribozymes (Steinecke, et al., EMBO J., 11:1525 (1992); Perriman, et al., Antisense Res. Dev., 3:253 (1993)); oligonucleotide mediated targeted modification (e.g., U.S. Pat. Nos. 6,528,700 and 6,911,575); Zn-finger targeted molecules (e.g., U.S. Pat. Nos. 7,151,201, 6,453,242, 6,785,613, 7,177,766 and 7,788,044); transposable elements (e.g. Dubin, M.J., et al., Transposons: a blessing curse, Current opinion in plant biology, Vol: 42, Page: 23-29, 2018 and Eric T. Johnson, Jesse B. Owens & Stefan Moisyadi (2016) Vast potential for using the piggyBac transposon to engineer transgenic plants at specific genomic locations, Bioengineered, 7:1, 3-6) and other methods or combinations of the above methods known to those of skill in the art.

The foregoing methods for transformation may be used for producing a transgenic variety. The transgenic variety could then be crossed with another (non-transformed or transformed) variety in order to produce a new transgenic variety. Alternatively, a genetic trait that has been engineered into a particular *Calibrachoa* variety using the foregoing transformation techniques could be moved into another variety using traditional backcrossing techniques that are well known in the plant breeding arts. For example, a backcrossing approach could be used to move an engineered trait from a public, non-elite variety into an elite variety, or from a variety containing a foreign gene in its genome into a variety or varieties that do not contain that gene.

Likewise, plants can be genetically engineered to express various phenotypes of interest, including, but not limited to, genes that confer resistance to pests or disease, genes that confer resistance to an herbicide, genes that confer or contribute to a value-added or desired trait, genes that control male sterility, genes that create a site for site specific DNA integration, and genes that affect abiotic stress resistance. Non-limiting examples of particular genes and corresponding phenotypes one may choose to introduce into a plant include one or more genes for insect tolerance, such as a *Bacillus thuringiensis* (Bt.), pest tolerance such as genes for fungal disease control, herbicide tolerance such as genes conferring glyphosate tolerance, and genes for quality improvements such as, environmental or stress tolerances, or any desirable changes in plant physiology, growth, development, morphology or plant product(s). For example, *Bacillus* insect control protein gene as described in WO 99/31248, herein incorporated by reference in its entirety, U.S. Pat. No. 5,689,052, herein incorporated by reference in its entirety, U.S. Pat. Nos. 5,500,365 and 5,880,275, herein incorporated by reference in their entirety. The gene can confer tolerance to the herbicide glyphosate as conferred by genes including, but not limited to Agrobacterium strain CP4 glyphosate resistant EPSPS gene (aroA:CP4) as described in U.S. Pat. No. 5,633,435, herein incorporated by reference in its entirety, or glyphosate oxidoreductase gene (GOX) as described in U.S. Pat. No. 5,463,175, herein incorporated by reference in its entirety. Alternatively, the DNA coding sequences can affect these phenotypes by encoding a non-translatable RNA molecule that causes the targeted inhibition of expression of an endogenous gene, for example via antisense- or cosuppression-mediated mechanisms (see, for example, Bird et al., Biotech. Gen. Engin. Rev., 9:207, 1991). The RNA could also be a catalytic RNA molecule (i.e., a ribozyme) engineered to cleave a desired endogenous mRNA product (see for example, Gibson and Shillito, Mol. Biotech., 7:125, 1997). Thus, any sequence which produces a phenotype or morphology change of interest may be used with the double-flowering dwarf *Calibrachoa* plants disclosed herein.

### Tissue Culture

Further reproduction can occur by tissue culture and regeneration. Tissue culture of various tissues of ornamental plants and regeneration of plants therefrom is well-known and widely published. For example, reference may be had to Valla Rego, Luciana et al., Crop Breeding and Applied Technology. 1(3): 283-300 (2001); Komatsuda, T., et al., Crop Sci., 31:333-337 (1991); Stephens, P. A., et al., Theor. Appl. Genet., 82:633-635 (1991); Komatsuda, T., et al., Plant Cell, Tissue and Organ Culture, 28:103-113 (1992); Dhir, S., et al., Plant Cell Reports, 11 :285-289 (1992); Pandey, P., et al., Japan J. Breed., 42:1-5 (1992); and Shetty, K., et al., Plant Science, 81:245-251 (1992). Thus, cells can be provided which upon growth and differentiation produce *Calibrachoa* plants exhibiting, or carrying the alleles for, double-flowering and dwarf traits described in the present application.

Regeneration refers to the development of a plant from tissue culture. The term "tissue culture" indicates a composition comprising isolated cells of the same or a different type or a collection of such cells organized into parts of a plant. Exemplary types of tissue cultures are protoplasts, calli, plant clumps, and plant cells that can generate tissue culture that are intact in plants or parts of plants, such as embryos, pollen, flowers, seeds, pods, petioles, leaves, stems, roots, root tips, anthers, pistils, and the like. Means for preparing and maintaining plant tissue culture are well known in the art. By way of example, a tissue culture comprising organs has been used to produce regenerated plants. U.S. Pat. Nos. 5,959,185, 5,973,234, and 5,977,445 describe certain techniques.

### Production of Double Haploids

The production of double haploids can also be used for the development of plants with a homozygous phenotype in the breeding program. Double haploids are produced by the doubling of a set of chromosomes (1N) from a heterozygous plant to produce a completely homozygous individual. This can be advantageous because the process omits the generations of selfing needed to obtain a homozygous plant from a heterozygous source. For example, see, Ferrie, Alison M.R., et al., "Review of Doubled Haploidy Methodologies in Ornamental Species" Propagation of Ornamental Plants. 11(2): pp. 63-77 (2011).

### Protoplast Fusion

Also known as somatic fusion, this process can be used with the double-flowering dwarf *Calibrachoa* plants of the present disclosure to create hybrids. The resulting hybrid plants have the chromosomes of each parent and thus the process is useful for incorporating new traits. The protoplast fusion technique is well known in the art; see for example Hamill J.D., Cocking E.C. (1988) Somatic Hybridization of Plants and its Use in Agriculture. In: Pais M.S.S., Mavituna F., Novais J.M. (eds) Plant Cell Biotechnology. NATO ASI Series (Series H: Cell Biology), vol 18.

### Gene Editing Using CRISPR

Targeted gene editing can be done using CRISPR/Cas9 technology (Saunders & Joung, Nature Biotechnology, 32, 347-355, 2014). CRISPR is a type of genome editing system that stands for Clustered Regularly Interspaced Short Palindromic Repeats. This system and CRISPR-associated (Cas) genes enable organisms, such as select bacteria and archaea, to respond to and eliminate invading genetic material. Ishino, Y., et al. J. Bacteriol. 169, 5429-5433 (1987). These repeats were known as early as the 1980s in E. coli, but Barrangou and colleagues demonstrated that *S. thermophilus* can acquire resistance against a bacteriophage by integrating a fragment of a genome of an infectious virus into its CRISPR locus. Barrangou, R., et al. Science 315, 1709-1712 (2007). Many plants have already been modified using the CRISPR system, for example petunia, a close relative of *Calibrachoa.* See for example, Zhang, B. et al., "Exploiting the CRISPR/Cas9 System for Targeted Genome Mutagenesis in Petunia" Science Reports, Vol. 6, February 2016.

Gene editing can also be done using crRNA-guided surveillance systems for gene editing. Additional information about crRNA-guided surveillance complex systems for gene editing can be found in the following documents, which are incorporated by reference in their entirety: U.S. Application Publication No. 2010/0076057 (Sontheimer et al., Target DNA Interference with crRNA); U.S. Application Publication No. 2014/0179006 (Feng, CRISPR-CAS Component Systems, Methods, and Compositions for Sequence Manipulation); U.S. Application Publication No. 2014/0294773 (Brouns et al., Modified Cascade Ribonucleoproteins and Uses Thereof); Sorek et al., Annu. Rev. Biochem. 82:237-266, 2013; and Wang, S. et al., Plant Cell Rep (2015) 34: 1473-1476.

### Gene editing using TALENs

Transcription activator-like effector nucleases (TALENs) have been successfully used to introduce targeted mutations via repair of double stranded breaks (DSBs) either through non-homologous end joining (NHEJ), or by homology-directed repair (HDR) and homology-independent repair in the presence of a donor template. Thus, TALENs are another mechanism for targeted genome editing in double-flowering dwarf *Calibrachoa* plants. The technique is well known in the art; see for example Malzahn, Aimee et al. "Plant genome editing with TALEN and CRISPR" Cell & Bioscience vol. 7 21. 24 Apr. 2017.

### Other methods of genome editing

In addition to CRISPR and TALENs, two other types of engineered nucleases can be used for genome editing: engineered homing endonucleases/meganucleases (EMNs), and zinc finger nucleases (ZFNs). These methods are well known in the art. See for example, Petilino, Joseph F. "Genome editing in plants via designed zinc finger nucleases" In Vitro Cell Dev Biol Plant. 51(1): pp. 1-8 (2015); and Daboussi, Fayza, et al. "Engineering Meganuclease for Precise Plant Genome Modification" in Advances in New Technology for Targeted Modification of Plant Genomes. Springer Science+Business. pp 21-38 (2015).

### EXAMPLES

The following examples are provided to illustrate further the various applications and are not intended to limit the disclosure beyond the limitations set forth in the appended claims.

### Example 1: Breeding with purple double-flowering and dwarf traits

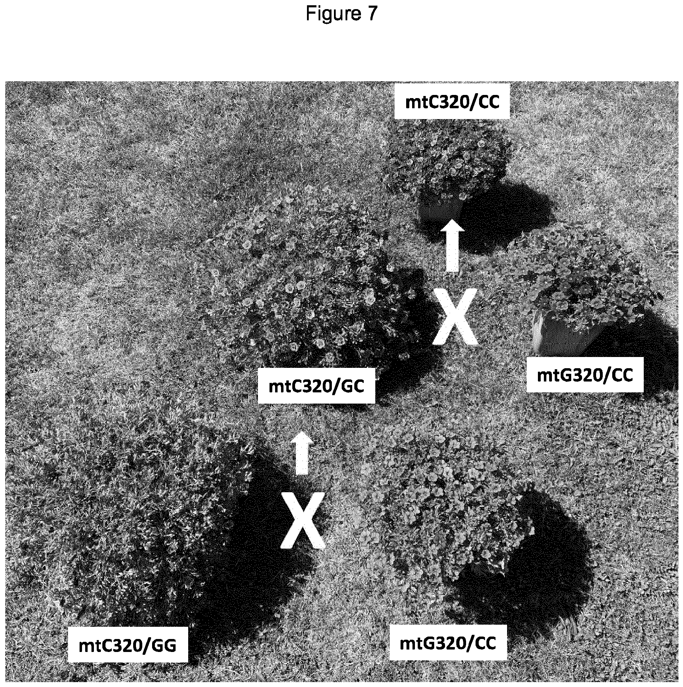

*Calibrachoa* plants having both the double-flowering and dwarf traits were bred using the molecular markers disclosed herein. As shown in Figure 7, a purple plant having double-flowers (mtC320) and normal vigorous growth (G/G) was used as the female parent and crossed with a purple dwarf plant (C/C) having single flowers (mtG320) as the male parent. The F₁ progeny exhibited purple double-flowers (mtC320) and normal vigorous growth as it was heterozygous for the recessive dwarf allele (G/C). This F₁ progeny was used a female parent in a cross with a male purple dwarf plant (C/C) having single flowers (mtG320). While an outcrossing is shown in Figure 7, those skilled in the art will understand that this progeny could also be backcrossed to the first male parent. F₂ progeny were genotyped for the molecular markers disclosed herein using methods well known in the art. A selected F₂ progeny from this cross is shown at the top of Figure 7, a purple double-flowering (mtC320) dwarf (C/C) *Calibrachoa.*

### Example 2: Breeding with white double-flowering and dwarf traits

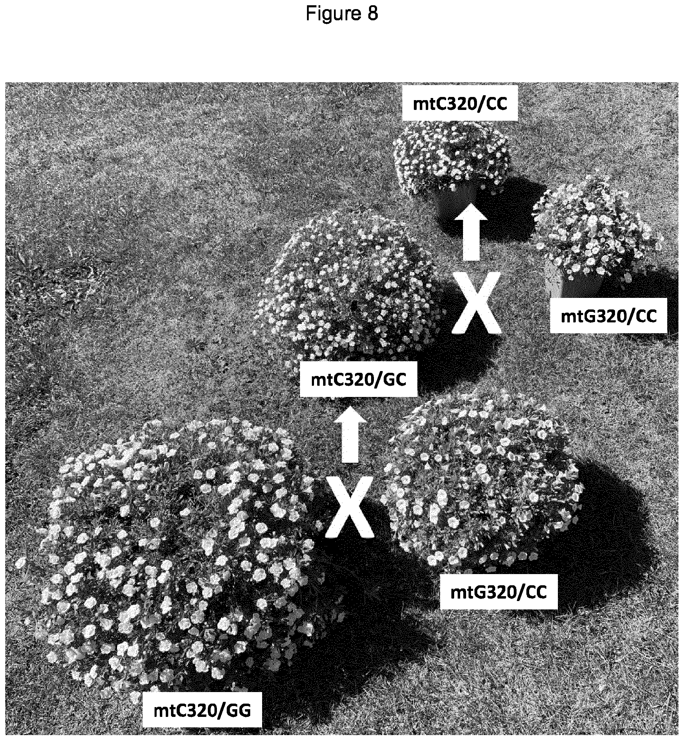

As shown in Figure 8, a white plant having double-flowers (mtC320) and normal vigorous growth (G/G) was used as the female parent and crossed with a white dwarf (C/C) plant having single flowers (mtG320) as the male parent. The F₁ progeny exhibited white double-flowers (mtC320) and normal vigorous growth as it was heterozygous for the recessive dwarf allele (G/C). This F₁ progeny was used a female parent in a cross with a male white dwarf (C/C) plant having single flowers (mtG320). While an outcrossing is shown in Figure 8, those skilled in the art will understand that this progeny could also be backcrossed to the first male parent. F₂ progeny were genotyped for the molecular markers disclosed herein using methods well known in the art. A selected F₂ progeny from this cross is shown at the top of Figure 8, a white double-flowering (mtC320) dwarf (C/C) *Calibrachoa.*

### Example 3: Double-flowering varieties with and without the dwarf trait

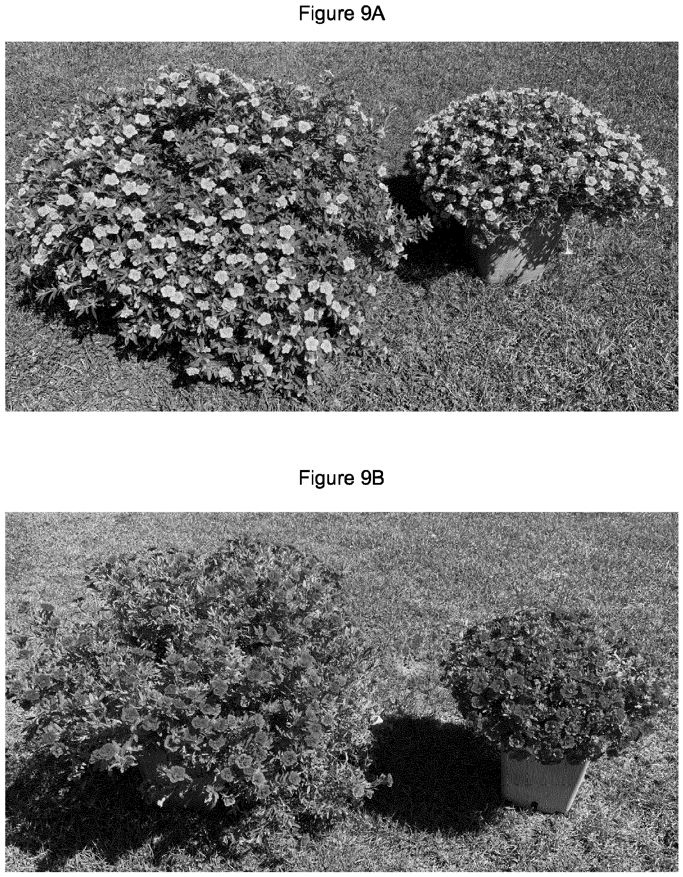

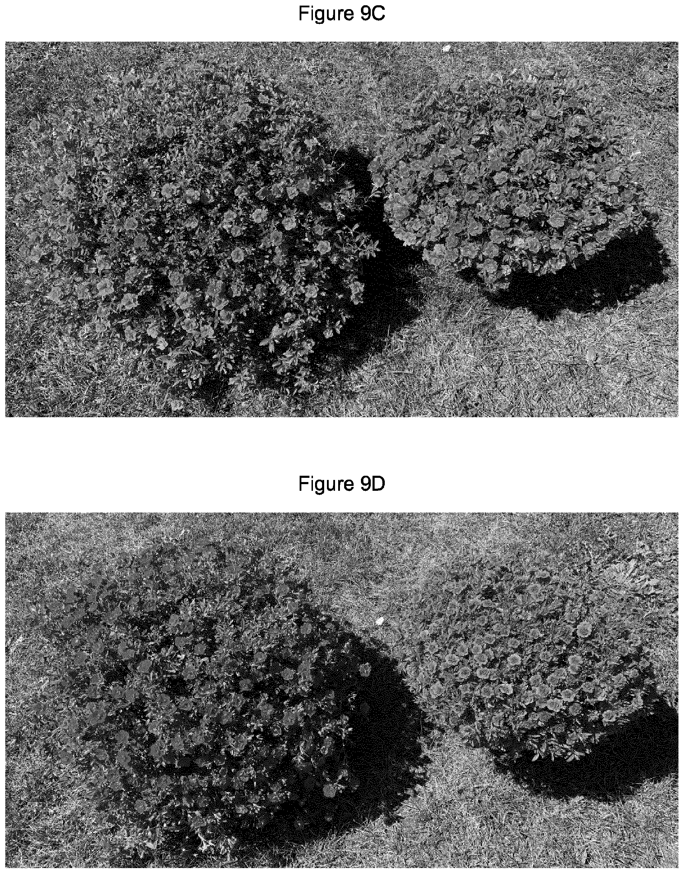

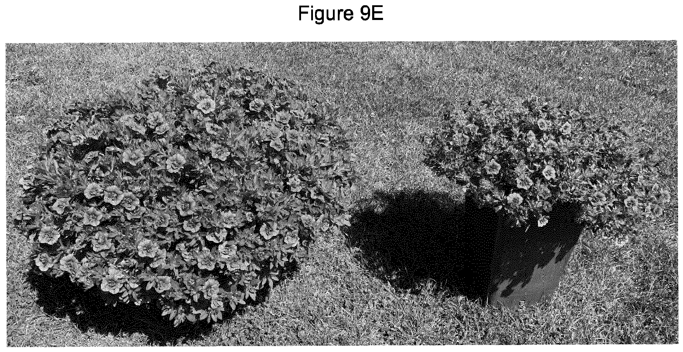

Figures 9A-9G show plants of different coloured varieties all having the double-flowering trait combined with either a wild-type allele for the dwarf trait (plant pictured left, G/G or G/C), or homozygous for the recessive dwarf allele (plant pictured right) associated with the nucleotide polymorphism shown in Figure 4A (C/C). Plants were bred using the breeding methods and schemes disclosed herein. Shown in Figure 9A are plants of yellow varieties. Shown in Figure 9B are plants of red varieties. Shown in Figure 9C are plants of pink varieties. Shown in Figure 9D are plants of pink-purple varieties, and shown in Figure 9E are plants of purple-red varieties.

### Example 4: White, yellow, and orange double-flowering dwarf Calibrachoa varieties generated using the methods disclosed herein

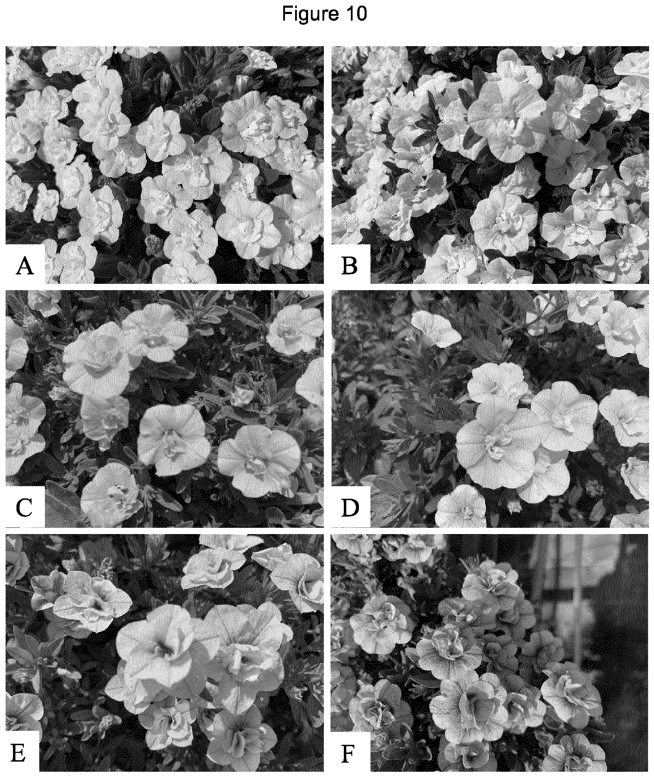

Figures 10A-10F are close up photographs of double-flowering dwarf *Calibrachoa* plants of the present disclosure having varying shades of white coloured flowers (Figure 10A and 10B), yellow coloured flowers (Figure 10C and 10D) and yellow-orange coloured flowers (Figure 10E and 10F). Shown in Figure 10A and 10B are plants of white varieties designated CA-2020-0723 and CA-2020-0710 respectively. Shown in Figure 10C is a plant of a yellow variety designated CA-2020-0743. Shown in Figure 10D is a plant of a light yellow variety having a large area of yellow at the transition to the corolla tube designated CA-2020-0735. Shown in Figure 10E is a plant of a light orange variety having strong red veins, designated CA-2020-0611, and shown in Figure 10F is a plant of a yellow-orange variety having strong red veins designated CA-20202-0931.

### Example 5: Double-flowering dwarf Calibrachoa varieties in varying shades of red and orange generated using the methods disclosed herein

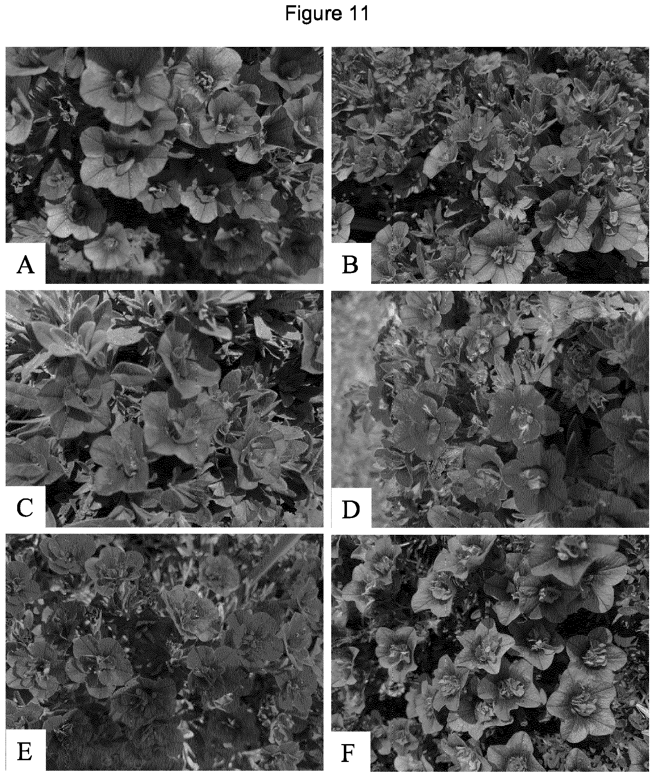

Figures 11A-11 F are close up photographs of double-flowering dwarf *Calibrachoa* plants of the present disclosure having varying shades of red and orange coloured flowers. Shown in Figures 11A and 11B are two plants of orange varieties, designated CA-2020-0825 and CA-2020-0833 respectively. Shown in Figure 11C (CA-2020-0810), Figure 11D (CA-2020-0805) and Figure 11E (CA-2020-0697) are three plants of red varieties, and Figure 11F shows a plant of a light red variety designated CA-2020-0809.

### Example 6: Double-flowering dwarf Calibrachoa varieties in varying shades of pink generated using the methods disclosed herein

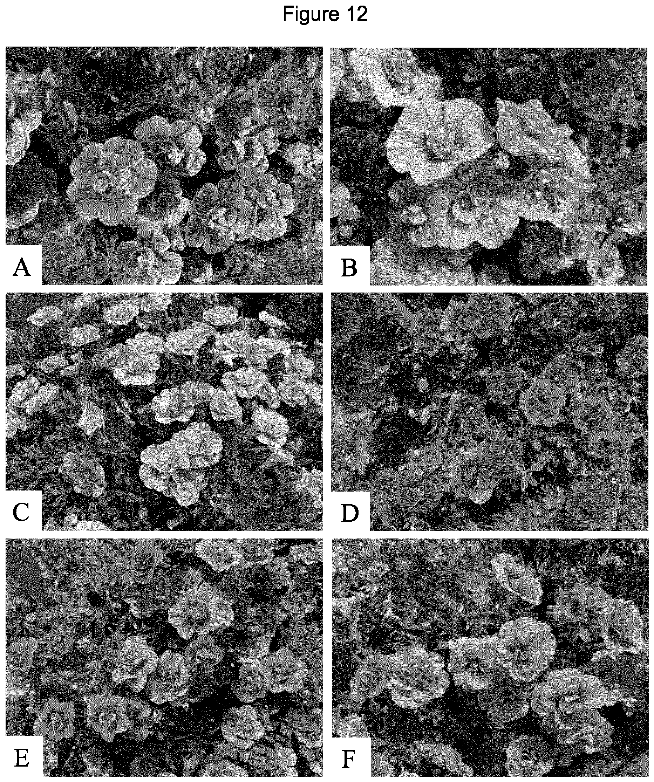

Figures 12A-12F, Figures 13A-13F, and Figures 14A-14D are close up photographs of double-flowering dwarf *Calibrachoa* plants of the present disclosure having varying shades of pink coloured flowers. In Figure 12A, a plant of a pink-purple variety is shown having a white colour at the margin of the corolla lobes and designated CA-2020-0766. Figures 12B and 12C show plants of two purple-pink varieties designated CA-2020-0604 and CA-2020-0748 respectively. In Figure 12D a plant of a dark pink-red variety having irregular magenta and yellow coloured petaloids, designated CA-2020-0775, is shown. Figures 12E and 12F show plants of two dark pink varieties having strong veins, designated CA-2020-0772 and CA-2020-0762 respectively.

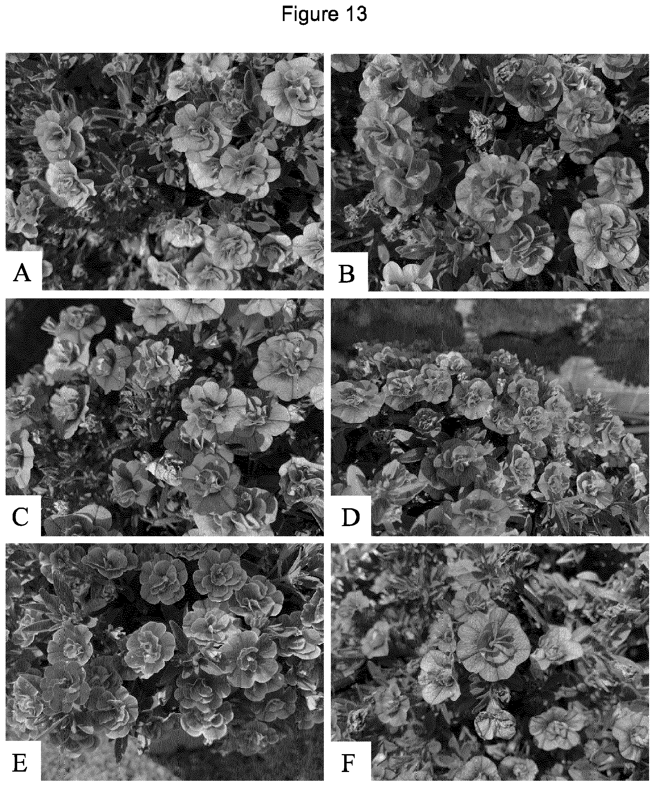

Figure 13A (CA-2020-0784), Figure 13B (CA-2020-0782) and Figure 13C (CA-2020-0780) show plants of three purple-pink varieties. Figure 13D shows a plant of a dark pink-red variety designated CA-2020-0778. In Figure 13E, a plant of variety CA-2019-5055 is shown having pink flowers with a white colour at the margin of the corolla lobes, and Figure 13F shows a plant of a pink variety designated CA-2019-4954.

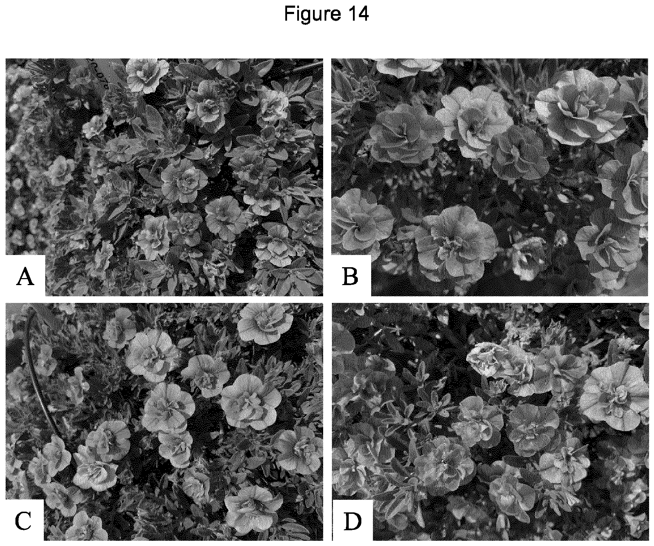

Figures 14A and 14B show plants of two purple-red varieties designated CA-2020-0763 and CA-2020-0755 respectively. Figure 14C shows plants of a dark pink-red variety designated CA-2020-0788 and Figure 14D shows a plant of a purple-pink variety designated CA-2020-0797.

### Example 7: Double-flowering dwarf Calibrachoa varieties in varying shades of purple generated using the methods disclosed herein

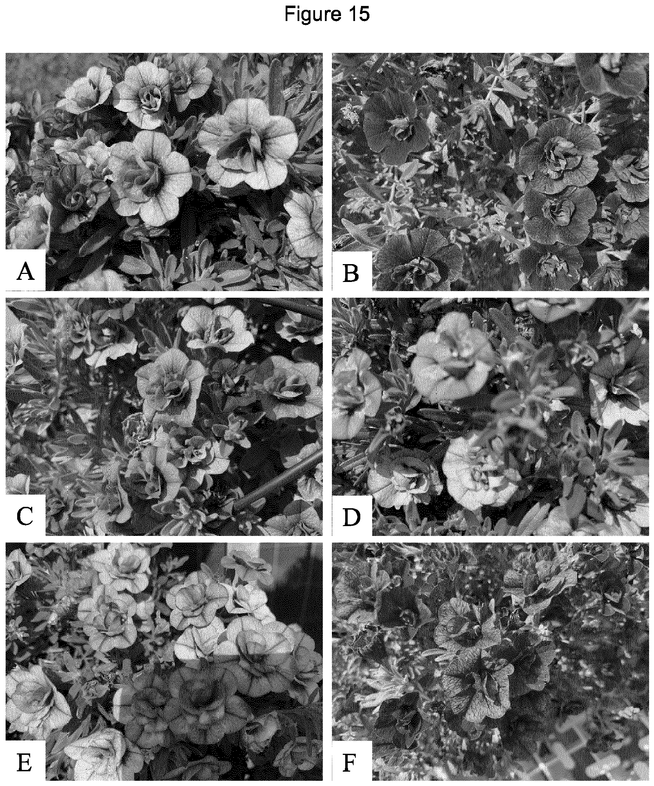

Figures 15A-15F are close up photographs of double-flowering dwarf *Calibrachoa* plants of the present disclosure having varying shades of purple coloured flowers. Figures 15A and 15B show plants of two dark pink-violet varieties having strong veins, designated CA-2020-0842 and CA-2020-0708 respectively. Figure 15C shows a plant of a brown-orange variety with strong purple veins designated CA-2020-0837. Figure 15D shows a plant of a light violet variety having medium violet veins, designated CA-2020-0843. Figure 15E shows a plant of a violet variety designated CA-2020-0846, and Figure 15F shows a plant of a purple variety with strong veins, designated CA-2019-5258.

### Example 8: Double-flowering dwarf Calibrachoa varieties in varying colours and patterns generated using the methods disclosed herein

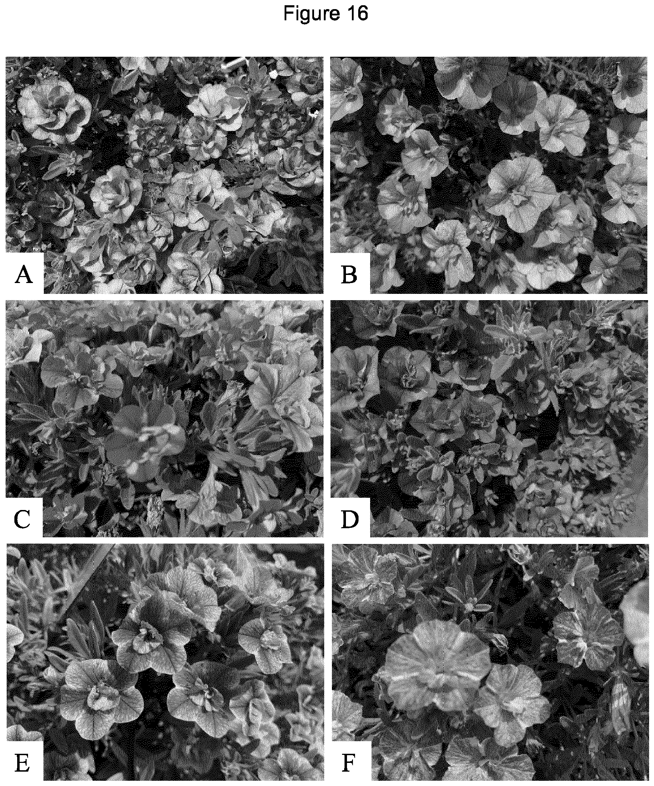

Figures 16A-16F are close up photographs of double-flowering dwarf *Calibrachoa* plants of the present disclosure having varying colours and patterns. Figure 16A shows a plant of a variety designated CA-2019-5264 having light purple flowers with a darker purple colour at the distal part of the corolla lobes. Figure 16B shows a plant of a variety designated CA-2020-0900 having red-pink flowers with a broad yellow colour along the fused parts of the corolla lobes. Figure 16C shows a plant of a variety designated CA-2020-0765 having pink-purple flowers with irregular light-pink colour distribution. Figure 16D shows a plant of a variety designated CA-2020-0812 having orange-red flowers with irregular yellow colour distribution. Figure 16E shows a plant of a variety designated CA-2020-0680 having pink-purple flowers with a small area of black at the transition to the corolla tube and a white margin on the corolla lobes. Figure 16F shows a plant of a variety designated CA-2020-0589 having pink-purple flowers with irregular white colour distribution.

### Example 9: Double-flowering dwarf Calibrachoa varieties in varying colours with contrasting veins generated using the methods disclosed herein

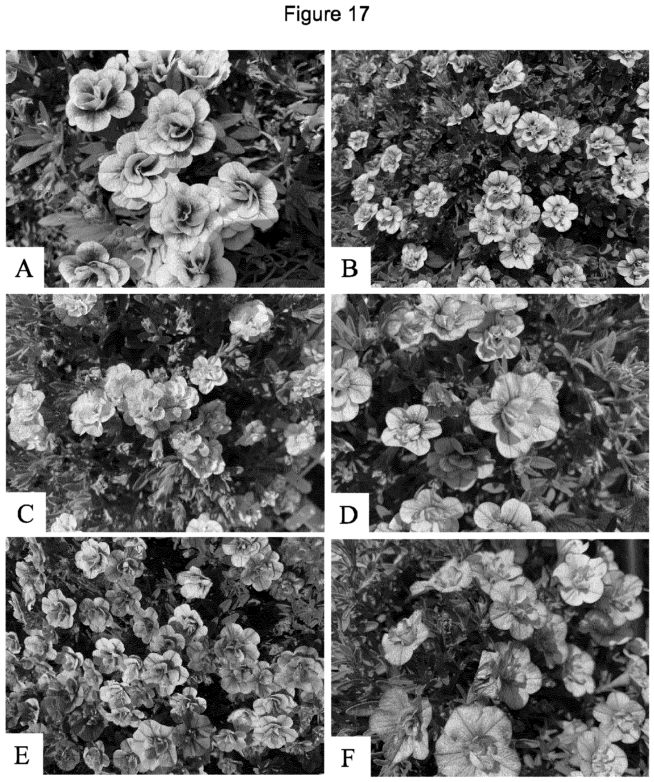

Figures 17A-17F are close up photographs of double-flowering dwarf *Calibrachoa* plants of the present disclosure having different coloured flowers with contrasting veins. Shown in Figure 17A is a plant of a purple variety with white margins on the corolla lobes and dark purple veins, designated CA-2020-5194. Shown in Figure 17B is a plant of a light yellow-orange variety with strong red veins, designated CA-2020-0773. Shown in Figure 17C is a plant of a light yellow variety with very strong purple veins, designated CA-2016-8659. Shown in Figure 17D is a plant of a light yellow variety with very strong red veins, designated CA-2020-0612. In Figure 17E, a plant of a purple-red variety with strong pink veins designated CA-2020-0790 is shown, and in Figure 17F, a plant of variety designated CA-2020-0923 having light yellow flowers with strong red veins and a red colour at the margin of corolla tubes is shown.

### Example 10: Double-flowering dwarf Calibrachoa varieties in varying colours with variations of the star pattern generated using the methods disclosed herein

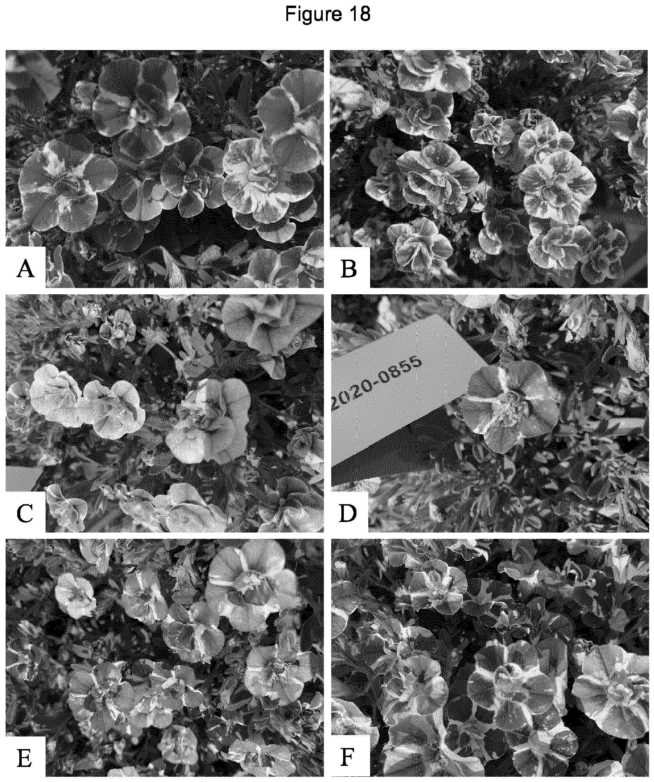

Figures 18A-18F are close up photographs of double-flowering dwarf *Calibrachoa* plants of the present disclosure having different coloured flowers and variations of the star pattern. Shown in Figures 18A and 18B are plants of two varieties, designated CA-2020-0579 and CA-2020-0581 respectively, having pink-purple flowers with very large yellow markings at the transition to the corolla tube and a white colour along the fused parts of the corolla lobes. In Figures 18C and 18D, plants of violet and dark violet varieties designated CA-2020-0854 and CA-2020-0855 respectively are shown having a white colour along the fused parts of the corolla lobes. Figures 18E and 18F show plants of two varieties, designated CA-2020-0856 and CA-2020-0857 respectively, having dark pink-violet flowers with a white colour along the fused parts of the corolla lobes.

### Example 11: Double-flowering dwarf Calibrachoa varieties in varying shades of pink-purple with the star pattern generated using the methods disclosed herein

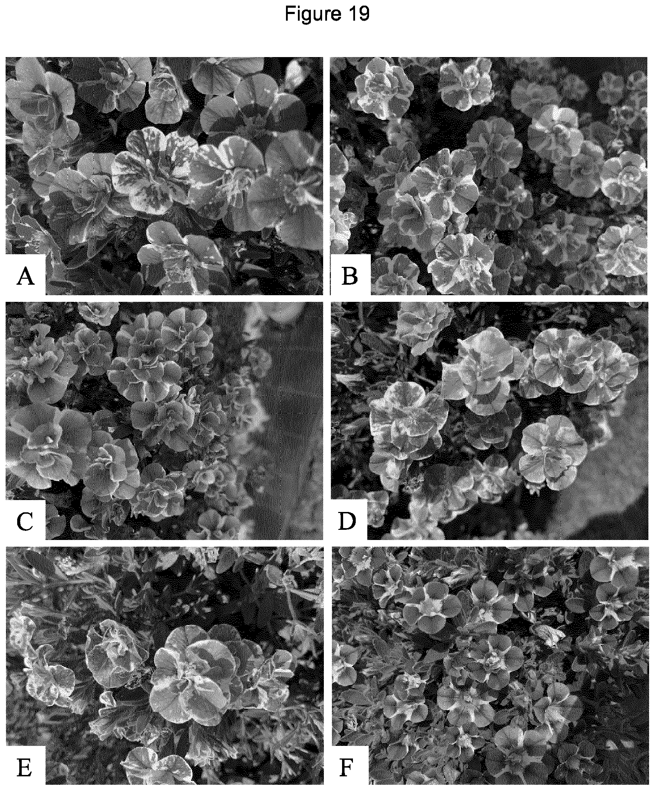

Figures 19A-19F are close up photographs of double-flowering dwarf *Calibrachoa* plants of the present disclosure in varying shades of pink-purple with the star pattern, all having some degree of white colouration along the fused parts of the corolla lobes. Figure 19A shows a plant of variety designated CA-2020-0862, which also exhibits some irregular white colour distribution in addition to the star pattern. Figure 19B shows a plant of variety designated CA-2020-0864. Figure 19C and 19D show plants of varieties designated CA-2020-0870 and CA-2020-0883, respectively, and Figure 19E shows a plant of a variety designated CA-2020-0875. Figure 19F shows a plant of a variety designated CA-2020-0889 having a milder double-flowering phenotype, with an additional darker colour at the transition to the corolla tube.

### Example 12: Double-flowering dwarf Calibrachoa varieties in different colours with variations of the star pattern generated using the methods disclosed herein

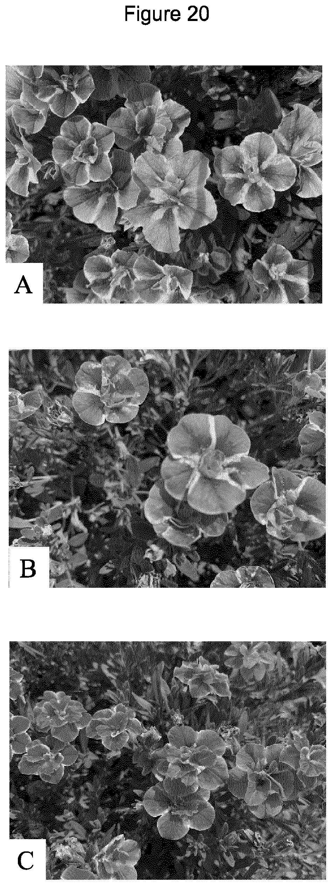

Figures 20A-20D are close up photographs of double-flowering dwarf *Calibrachoa* plants of the present disclosure having different coloured flowers and variations of the star pattern. A plant of a red variety having yellow along the fused parts of the corolla lobes, designated CA-2019-5092 is shown in Figure 20A. A plant of a purple-pink variety designated CA-2020-0678 with medium white colour along the fused parts of the corolla lobes is shown in Figure 20B. Figure 20C shows a plant of a variety designated CA-2020-0750 having purple-pink flowers with white margins of the corolla lobes.

### Example 13: Double-flowering dwarf Calibrachoa varieties in different colours with variations of white margins generated using the methods disclosed herein

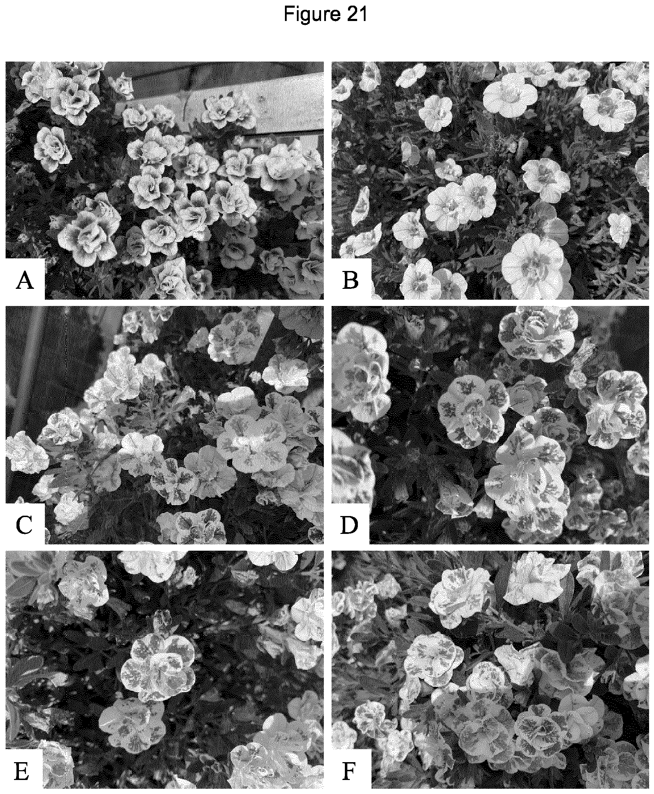

Figures 21A-21F are close up photographs of double-flowering dwarf *Calibrachoa* plants of the present disclosure having different coloured flowers and varying amounts of white colouration at the distal portions of the corolla lobes. Shown in Figure 21A is a plant of a purple variety designated CA-2017-1140 having a white margin of the corolla lobes. Figure 21B shows a plant of a variety designated CA-2020-0907 having purple-pink flowers with white at the distal part of the corolla tubes. Figures 21C shows a plant of a violet variety designated CA-2020-0899 having a white colour at the margin of the corolla lobes. Figures 21D-21F show plants of purple-pink varieties having a white colour at the margin of the corolla lobes designated CA-2020-0868, CA-2020-0869, and CA-2020-0894, respectively.

### Example 14: Generating a double-flowering and/or dwarf Calibrachoa via a gene editing tool or technology

In the event that the SNP mutations disclosed herein are the causative SNP mutations for the double-flowering and/or dwarf trait, single flowering vigorous *Calibrachoa* plants may be converted to a double-flowering and/or dwarf phenotype by targeted genetic engineering. Such methods may include, for example, the CRISPR system. Many plants have already been modified using the CRISPR system, for example *Petunia,* a close relative of *Calibrachoa.* See for example, Zhang, B. et al., "Exploiting the CRISPR/Cas9 System for Targeted Genome Mutagenesis in Petunia" Science Reports, Vol. 6, February 2016.

Transcription activator-like effector nucleases (TALENs) have been successfully used to introduce targeted mutations via repair of double stranded breaks (DSBs) either through non-homologous end joining (NHEJ), or by homology-directed repair (HDR) and homology-independent repair in the presence of a donor template. Thus, TALENs are another mechanism for targeted genome editing in *Calibrachoa.* The technique is well known in the art; see for example Malzahn, Aimee et al. "Plant genome editing with TALEN and CRISPR" Cell & Bioscience vol. 7 21. 24 Apr. 2017.

In addition to CRISPR and TALENs, two other types of engineered nucleases can be used for genome editing: engineered homing endonucleases/meganucleases (EMNs), and zinc finger nucleases (ZFNs). These methods are well known in the art. See for example, Petilino, Joseph F. "Genome editing in plants via designed zinc finger nucleases" In Vitro Cell Dev Biol Plant. 51(1): pp. 1-8 (2015); and Daboussi, Fayza, et al. "Engineering Meganuclease for Precise Plant Genome Modification" in Advances in New Technology for Targeted Modification of Plant Genomes. Springer Science+Business. pp 21-38 (2015).

### Example 15: Double-flowering dwarf Petunia-Calibrachoa (Petchoa) hybrids

*Petunia* and *Calibrachoa* are closely related. In the 1990's, several species of *Petunia* were crossed with *Calibrachoa.* The resulting hybrid offspring was named *Petchoa.* The double-flowering dwarf *Calibrachoa* plants disclosed herein can be used in a plant breeding program to produce double-flowering dwarf *Petchoas.* For example, a female double-flowering dwarf *Calibrachoa* may be crossed with a male *Petunia* carrying at least one allele for a dwarf trait. Embryos may be rescued and cultured by techniques well known in the art, and the plant(s) can be grown and further propagated by tissue culture and cuttings.

### BRIEF DESCRIPTION OF THE SEQUENCE LISTINGS

SEQ ID NO: 1 is a sequence from the mitochondrial genome of *Calibrachoa* (SEQ ID NO: 7) identifying the location of two single nucleotide polymorphisms at positions 247 and 320.
SEQ ID NO: 2 is a sequence from the nuclear genome of *Calibrachoa* identifying the location a single nucleotide polymorphisms at position 43.
SEQ ID NO: 3 is a sequence from the mitochondrial genome of *Calibrachoa* comprising two single nucleotide polymorphisms at positions 247 and 320 associated with an allele for double-flowering.
SEQ ID NO: 4 is a wild-type (single flower) sequence from the mitochondrial genome of *Calibrachoa.*
SEQ ID NO: 5 is a sequence from the nuclear genome of *Calibrachoa* comprising a single nucleotide polymorphism at position 43.
SEQ ID NO: 6 is a wild-type (non-dwarf) sequence from the nuclear genome of *Calibrachoa.*
SEQ ID NO: 7 is a whole mitochondrial genome sequence from *Calibrachoa,* and shows the single nucleotide polymorphisms of SEQ ID NO: 1, here at positions 224,919 and 224,992.

## Claims

1. A molecular marker for distinguishing a plant having an allele for a double-flowering characteristic, said distinguishing comprising detecting at least one SNP mutation selected from the group consisting of:
1. a C nucleotide at position number 320 of SEQ ID NO: 1 and
2. a C nucleotide at position number 247 in SEQ ID NO: 1,
the marker comprising a sequence of at least 20 consecutive nucleotides of SEQ ID NO: 1, or the complementary sequence thereof, said molecular marker preferably being isolated.

2. A molecular marker for distinguishing a plant having at least one allele for a dwarf growth characteristic, said distinguishing comprising detecting a C nucleotide at position 43 of SEQ ID NO:2 , the marker comprising at least one sequence selected from the group consisting of SEQ ID NO: 2, cDNA sequences thereof, fragments of at least 20 consecutive nucleotides thereof, and complementary sequences thereof, said molecular marker preferably being isolated.

3. A method for distinguishing a plant having:
a) at least one allele for a double-flowering characteristic, comprising using the molecular marker of claim 1 and detecting at least one SNP mutation selected from the group consisting of:
1. a C nucleotide at position number 320 of SEQ ID NO: 1 and
2. a C nucleotide at position number 247 in SEQ ID NO: 1,
b) and/or at least one allele for a dwarf growth characteristic, comprising using the molecular marker of claim 2 and detecting a C nucleotide at position 43 of SEQ ID NO:2.

## Patentansprüche

1. Molekularer Marker zur Unterscheidung einer Pflanze mit einem Allel für ein Doppelblütenmerkmal, wobei die Unterscheidung den Nachweis mindestens einer SNP-Mutation umfasst, die aus der Gruppe ausgewählt ist, die besteht aus:
1. einem C-Nukleotid an Position Nummer 320 von SEQ ID NO: 1 und
2. einem C-Nukleotid an der Position Nummer 247 in SEQ ID NO: 1,
wobei der Marker eine Sequenz von mindestens 20 aufeinanderfolgenden Nukleotiden der SEQ ID NO: 1 oder die komplementäre Sequenz davon umfasst, wobei der molekulare Marker vorzugsweise isoliert ist.

2. Molekularer Marker zur Unterscheidung einer Pflanze mit mindestens einem Allel für ein Zwergwuchsmerkmal, wobei die Unterscheidung den Nachweis eines C-Nukleotids an Position 43 von SEQ ID NO: 2 umfasst, wobei der Marker mindestens eine Sequenz umfasst, die aus der Gruppe ausgewählt ist, die aus SEQ ID NO: 2, cDNA-Sequenzen davon, Fragmenten von mindestens 20 aufeinanderfolgenden Nukleotiden davon und komplementären Sequenzen davon besteht, wobei der molekulare Marker vorzugsweise isoliert ist.

3. Verfahren zur Unterscheidung einer Pflanze mit:
a) mindestens ein Allel für ein Doppelblütenmerkmal, umfassend die Verwendung des molekularen Markers nach Anspruch 1 und den Nachweis mindestens einer SNP-Mutation, ausgewählt aus der Gruppe bestehend aus:
1. einem C-Nukleotid an Position Nummer 320 von SEQ ID NO: 1 und
2. einem C-Nukleotid an der Position Nummer 247 in SEQ ID NO: 1,
b) und/oder mindestens ein Allel für ein Zwergwuchsmerkmal, umfassend die Verwendung des molekularen Markers nach Anspruch 2 und den Nachweis eines C-Nukleotids an Position 43 in SEQ ID NO: 2.

## Revendications

1. Marqueur moléculaire permettant de distinguer une plante présentant un allèle pour une caractéristique de double floraison, ladite distinction comprenant la détection d'au moins une mutation SNP choisie dans le groupe consistant en :
1. un nucléotide C à la position 320 de SEQ ID NO : 1 et
2. un nucléotide C à la position 247 de SEQ ID NO : 1,
le marqueur comprenant une séquence d'au moins 20 nucléotides consécutifs de SEQ ID NO: 1, ou la séquence complémentaire de celle-ci, ledit marqueur moléculaire étant de préférence isolé.

2. Marqueur moléculaire permettant de distinguer une plante présentant au moins un allèle pour une caractéristique de croissance naine, cette distinction consistant à détecter un nucléotide C en position 43 de SEQ ID NO:2, le marqueur comprenant au moins une séquence choisie dans le groupe constitué de SEQ ID NO : 2, de ses séquences d'ADNc, de ses fragments d'au moins 20 nucléotides consécutifs, et de ses séquences complémentaires, ce marqueur moléculaire étant de préférence isolé.

3. Méthode permettant de distinguer une plante ayant
a) au moins un allèle pour un caractère de double floraison, comprenant l'utilisation du marqueur moléculaire de la revendication 1 et la détection d'au moins une mutation SNP choisie dans le groupe constitué de :
1. un nucléotide C à la position 320 de SEQ ID NO : 1 et
2. un nucléotide C à la position 247 de SEQ ID NO : 1,
b) et/ou au moins un allèle pour une caractéristique de croissance naine, comprenant l'utilisation du marqueur moléculaire de la revendication 2 et la détection d'un nucléotide C à la position 43 de SEQ ID NO:2.
